(19) Europäisches Patentamt
European Patent Office
Office européen des brevets

(11) **EP 2 790 733 B1**

(12) **EUROPEAN PATENT SPECIFICATION**

(45) Date of publication and mention of the grant of the patent:
**30.10.2019 Bulletin 2019/44**

(21) Application number: **12820997.0**

(22) Date of filing: **14.12.2012**

(51) Int Cl.:
*A61K 9/51* (2006.01)  *A61K 9/00* (2006.01)
*A61K 31/12* (2006.01)  *A61K 31/7088* (2006.01)

(86) International application number:
**PCT/US2012/069882**

(87) International publication number:
**WO 2013/090804 (20.06.2013 Gazette 2013/25)**

(54) **NANOPARTICLES WITH ENHANCED MUCOSAL PENETRATION OR DECREASED INFLAMMATION**

NANOPARTIKEL MIT VERBESSERTER MUKOSALER PENETRATION ODER VERRINGERTER ENTZÜNDUNG

NANOPARTICULES PRÉSENTANT UNE PÉNÉTRATION MUCOSALE ACCRUE OU UNE INFLAMMATION DIMINUÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.12.2011 US 201161570405 P**
**14.12.2011 US 201161570413 P**
**08.02.2012 PCT/US2012/024344**

(43) Date of publication of application:
**22.10.2014 Bulletin 2014/43**

(73) Proprietor: **The Johns Hopkins University Baltimore, MD 21218 (US)**

(72) Inventors:
• **HANES, Justin**
**Baltimore, MD 21212 (US)**
• **XU, Qingguo**
**Baltimore, MD 21210 (US)**
• **BOYLAN, Nicholas**
**Baltimore, MD 21211 (US)**

(74) Representative: **Potter Clarkson**
**The Belgrave Centre**
**Talbot Street**
**Nottingham NG1 5GG (GB)**

(56) References cited:
**US-A1- 2008 166 414**

• GREF R ET AL: "'STEALTH' CORONA-CORE NANOPARTICLES SURFACE MODIFIED BY POLYETHYLENE GLYCOL (PEG): INFLUENCES OF THE CORONA (PEG CHAIN LENGTH AND SURFACE DENSITY) AND OF THE CORE COMPOSITION ON PHAGOCYTIC UPTAKE AND PLASMA PROTEIN ADSORPTION", COLLOIDS AND SURFACES. B, BIOINTERFACES, ELSEVIER, AMSTERDAM, NL, vol. 18, no. 3/04, 1 January 2000 (2000-01-01), pages 301-313, XP009061728, ISSN: 0927-7765, DOI: 10.1016/S0927-7765(99)00156-3
• YAN SHENG ET AL: "In vitro macrophage uptake and in vivo biodistribution of PLA-PEG nanoparticles loaded with hemoglobin as blood substitutes: effect of PEG content", JOURNAL OF MATERIALS SCIENCE: MATERIALS IN MEDICINE, KLUWER ACADEMIC PUBLISHERS, BO, vol. 20, no. 9, 14 April 2009 (2009-04-14) , pages 1881-1891, XP019730960, ISSN: 1573-4838, DOI: 10.1007/S10856-009-3746-9
• CU AND SALTZMAN: "Controlled Surface Modification with Poly(ethylene)glycol Enhances Diffusion of PLGA Nanoparticles in Human Cervical Mucus", MOLECULAR PHARMACEUTICS, AMERICAN CHEMICAL SOCIETY, US, vol. 6, no. 1, 1 February 2009 (2009-02-01), pages 173-181, XP008162859, ISSN: 1543-8384, DOI: 10.1021/MP8001254 [retrieved on 2008-11-20]

**(Cont. next page)**

- **Fred Reynolds ET AL: "Method of Determining Nanoparticle Core Weight", Analytical Chemistry, vol. 77, no. 3, 1 February 2005 (2005-02-01), pages 814-817, XP55448719, US ISSN: 0003-2700, DOI: 10.1021/ac049307x**
- **AUGUSTE D T ET AL: "pH triggered release of protective poly(ethylene glycol)-b-polycation copolymers from liposomes", BIOMATERIALS, ELSEVIER SCIENCE PUBLISHERS BV., BARKING, GB, vol. 27, no. 12, 1 April 2006 (2006-04-01) , pages 2599-2608, XP027951077, ISSN: 0142-9612 [retrieved on 2006-04-01]**
- **FAHMY T M ET AL: "Surface modification of biodegradable polyesters with fatty acid conjugates for improved drug targeting", BIOMATERIALS, ELSEVIER SCIENCE PUBLISHERS BV., BARKING, GB, vol. 26, no. 28, 1 October 2005 (2005-10-01), pages 5727-5736, XP027767526, ISSN: 0142-9612 [retrieved on 2005-10-01]**

Remarks:

The file contains technical information submitted after the application was filed and not included in this specification

**Description**

**FIELD OF THE INVENTION**

This invention is in the field of nanoparticles, particularly nanoparticles that rapidly penetrate mucus, such as human mucus, and methods of making and using thereof.

**BACKGROUND OF THE INVENTION**

Localized delivery of therapeutics via biodegradable nanoparticles often provides advantages over systemic drug administration, including reduced systemic side effects and controlled drug levels at target sites. Gref et al (2000) Colloids and Surfaces B: Biointerfaces, 18: 301-313 describes 'stealth' corona-core nanoparticles modified by polyethylene glycol (PEG) and discusses the influence of the corona (PEG chain length and surface density) and of the core composition on phagocytic uptake and plasma protein adsorption.

However, controlled drug delivery at mucosal surfaces has been limited by the presence of the protective mucus layer.

Mucus is a viscoelastic gel that coats all exposed epithelial surfaces not covered by skin, such as respiratory, gastrointestinal, nasopharyngeal, and female reproductive tracts, and the surface of eye. Mucus efficiently traps conventional particulate drug delivery systems via steric and/or adhesive interactions. As a result of mucus turnover, most therapeutics delivered locally to mucosal surfaces suffer from poor retention and distribution, which limits their efficacy. Biodegradable nanoparticles that penetrate deep into the mucus barrier can provide improved drug distribution, retention and efficacy at mucosal surfaces.

Dense coatings of low molecular weight polyethylene glycol (PEG) allow nanoparticles to rapidly penetrate through highly viscoelastic human mucus secretions. The hydrophilic and bioinert PEG coating effectively minimizes adhesive interactions between nanoparticles and mucus constituents. Biodegradable mucus-penetrating particles (MPPs) have been prepared by physical adsorption of certain PLURONICs, such as F127, onto pre-fabricated mucoadhesive nanoparticles. In addition, MPPs have been prepared by nanoprecipitation using diblock copolymers of poly(sebacic acid) and PEG.

However, the scope of biodegradable MPPs prepared by nanoprecipitation is limited because it requires dissolution of the drug and polymer in water-miscible solvents. Numerous hydrophobic drugs may benefit from local delivery by MPPs, due to serious systemic side effects, however their poor solubility in water-miscible organic solvents limits efficient encapsulation into MPPs by nanoprecipitation.

Other classes of emerging drugs, including nucleic acids, peptides and proteins, possess immense potential to treat diseases at mucosal sites. However, these hydrophilic drugs cannot be easily formulated into MPPs by nanoprecipitation. Emulsion solvent evaporation is widely used to efficiently encapsulate both hydrophilic drugs (water-in-oil-in-water double emulsion) and hydrophobic drugs (oil-in-water single emulsion) into biodegradable nanoparticles, however, the resulting particles are mucoadhesive when the conventional emulsifier, polyvinyl alcohol (PVA), is used. For example, Fahmy et al, 2005, Biomaterials, 26: 5727-5736 describes a modified water-in-oil-in-water emulsion method for the preparation of fatty acid PLGA particles in the presence of PVA ($M_w$ average 30-70 KDa) and avidin-palmitic acid. Cu and Saltzman, 2009, Mol. Pharm., 6(1): 173-181 described the production of PEG coated nanoparticles, using the PVA-containing particles of Fahmy et al, 2005 (*supra*) and binding thereto biotin conjugated to single amine-terminated PEG. Sheng et al, 2009, J. Mater. Sci: Mater Med, 20: 1881-1891, described PLA-PGE nanoparticles prepared from a primary emulsion formed with PVA.

There exists a need for new methods of preparing mucus-penetrating particles which can encapsulate a wide range of drugs into the nanoparticles without a decrease in the mucus penetrating properties as described above. There is a similar need for formulations which are administered via injection. It has been determined that similar coatings to those enhancing mucosal penetration also decrease inflammation elicited by the drug particles.

Therefore, it is an object of the invention to provide methods of preparing particles, and the resulting particles, which can encapsulate a wide range of drugs into the biodegradable nanoparticles without a decrease in the mucus penetrating properties or increase in the inflammation elicited by the particles as described above.

It is another object of the invention to provide particles, such as nanoparticles, with high drug loading and a dense coating of a surface-altering material to provide effective drug delivery via a variety of routes of administration.

**SUMMARY OF THE INVENTION**

Nanoparticles formed by emulsion of one or more core polymers, one or more surface altering materials, and one or more low molecular weight emulsifiers have been developed.

The present invention is defined by the appended claims.

More specifically, the present invention provides nanoparticles formed by emulsion of one or more core polymers, one or more surface altering materials, and one or more low molecular weight emulsifiers having a molecular weight less than 1500 amu, wherein the one or more surface altering materials are selected from the group consisting of polyethylene glycol (PEG) and poloxamer, or wherein the particles are formed from block copolymers containing PEG, wherein the nanoparticle possess a $\zeta$-potential of between 10 mV and -10 mV when dispersed in 10 mM NaCl solution at pH 7;
wherein the nanoparticles further comprise one or more therapeutic, prophylactic, or diagnostic agents, and
wherein the nanoparticles penetrate cervicovaginal mucus (CVM) with effective speeds less than 25-fold slower than the same particles in water.

The particles are made by dissolving the one or more core polymers in an organic solvent, adding the solution of the one or more core polymers to an aqueous solution or suspension of the emulsifier to form an emulsion, and then adding the emulsion to a second solution or suspension of the emulsifier to effect formation of the nanoparticles. The core and emulsifier can be separate, conjugated together, or in the form of a block copolymer containing one or more blocks of the surface altering material. In a preferred embodiment, the surface altering material is polyethylene glycol with a molecular weight from about 1kD to about 10kD, preferably from about 1kD to about 5kD, more preferably about 5kD. In this embodiment, the density of the polyethylene glycol, when measured by $^1$H NMR, is from about 1 to about 100 chains/nm$^2$, preferably from about 1 to about 50 chains/nm$^2$, more preferably from about 5 to about 50 chains/nm$^2$, most preferably from about 5 to about 25 chains/nm$^2$.

The criticality of the molecular weight of the emulsifiers is demonstrated by the examples. The molecular weight of the one or more emulsifiers is less than 1500 amu, and in a preferred embodiment less than 1300, 1200, 1000, 800, 600, or 500 amu. Preferred emulsifiers include cholic acid sodium salt, dioctyl sulfosuccinate sodium, hexadecyltrimethyl ammonium bromide, saponin, TWEEN® 20, TWEEN® 80, and sugar esters. The surface altering materials are present in an amount effective to make the surface charge of the particles neutral or essentially neutral when the one or more emulsifiers are charged. The emulsifiers have an emulsification capacity of at least about 50%, preferably at least 55, 60, 65, 70, 75, 80, 85, 90, or 95%.

The nanoparticles are particularly useful for the delivery of the therapeutic, prophylactic or diagnostic agents. These can be administered enterally, parenterally, or topically, preferably to a mucosal surface. In a preferred embodiment, the particles are administered by intravenous, subcutaneous, intramuscular, intraperitoneal injection, or subconjunctivally. In one embodiment the particles are administered to the pulmonary tract, intranasally, intravaginally, rectally, or buccally.

## BRIEF DESCRIPTION OF THE DRAWINGS

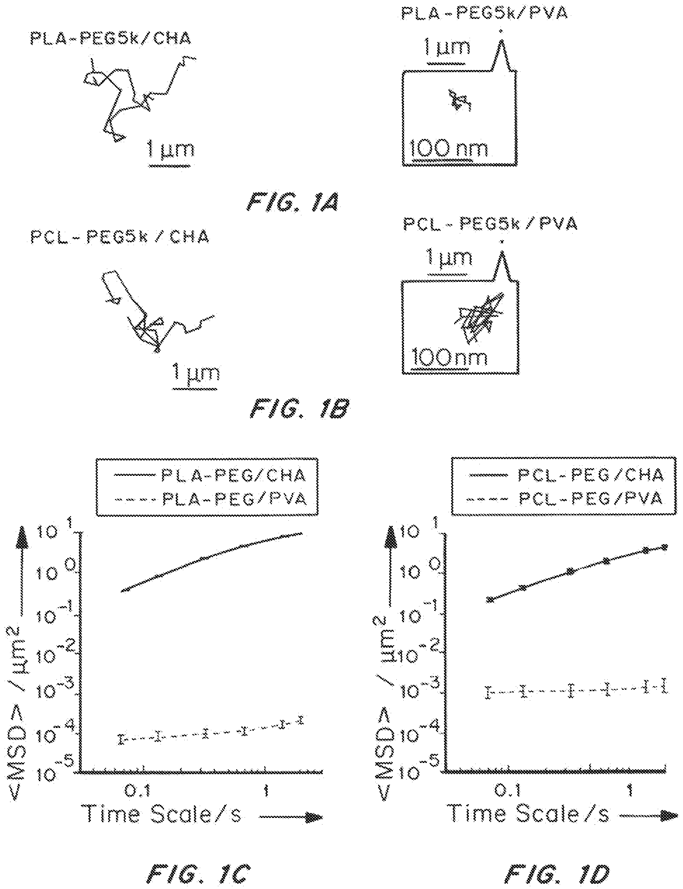

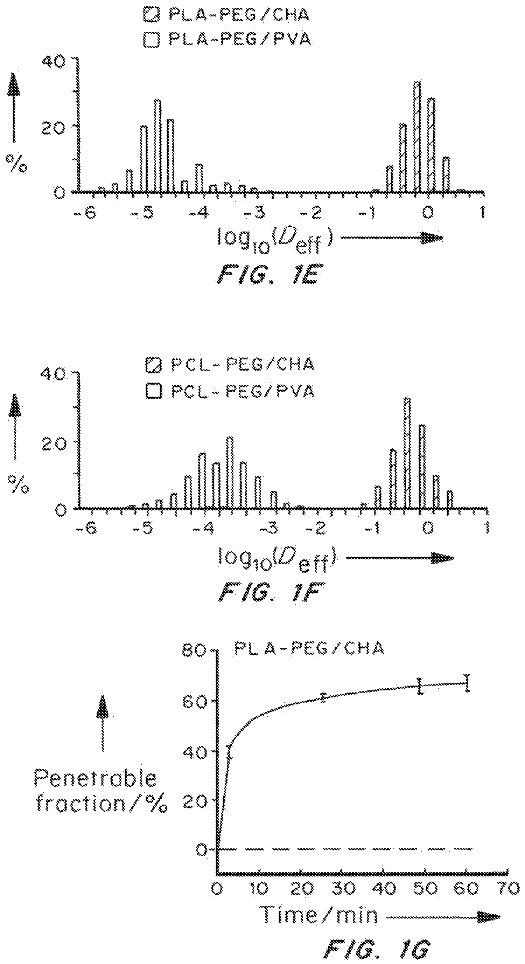

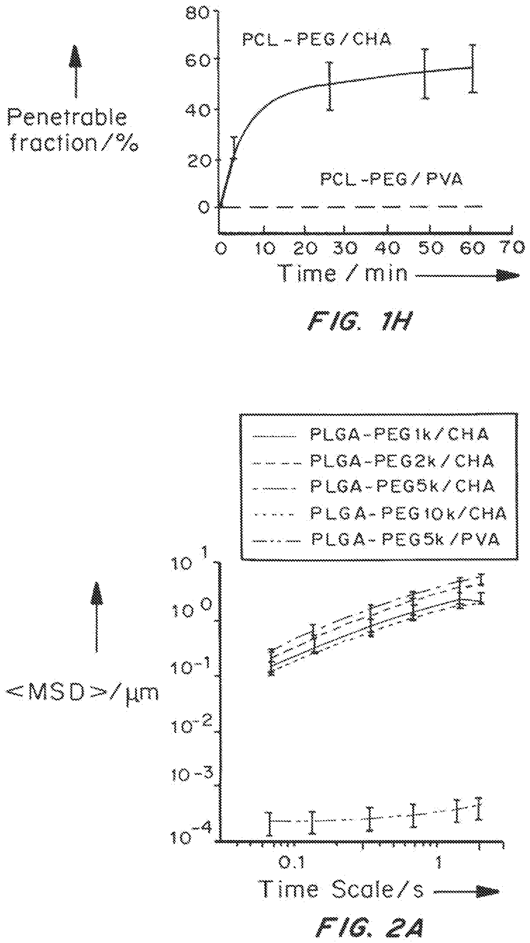

Figures 1a and 1b are representative trajectories of PLA-PEG and PCL-PEG nanoparticles containing CHA and PVA prepared by an emulsification method. Figures 1c and 1d are graphs showing the ensemble-averaged geometric mean square displacements (<MSD>/$\mu$m$^2$) as function of time (time scale/s). Figures 1e and 1f are graphs showing the distributions of the logarithms of individual particle effective diffusivities ($D_{eff}$) at a time scale of 1 s. Figures 1g and 1h are graphs showing the estimated fraction of particles capable of penetrating a physiological 30$\mu$m thick mucus layer over time. Data represent three independent experiments with $\geq$ 120 nanoparticles tracked for each experiment. Error bars are presented as s.e.m.

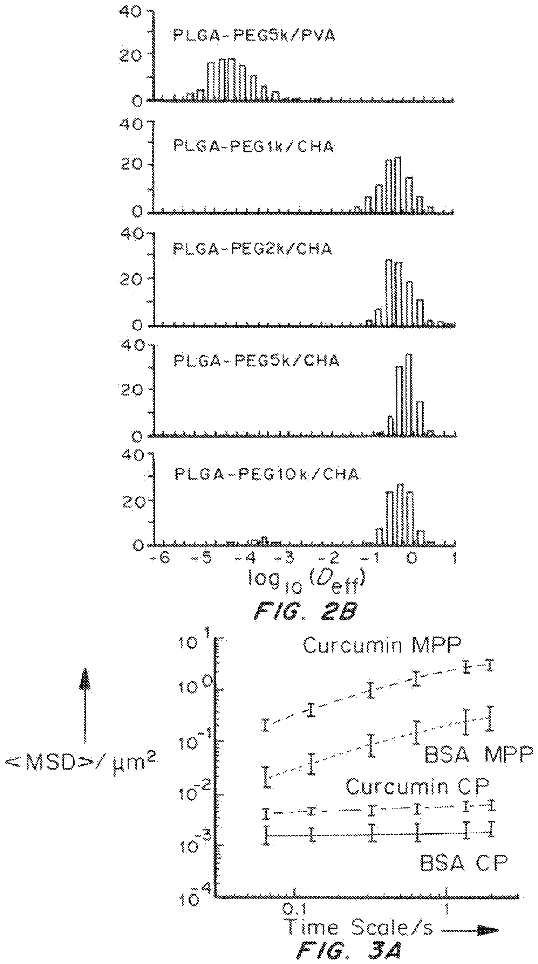

Figures 2a and 2b show the effect of PEG MW on transport rate of MPP in human cervicovaginal mucus: Figure 2a is a graph showing the ensemble-averaged geometric mean square displacement <MSD/ $\mu$m$^2$> as a function of time scale/s. Figure 2b is a graph showing the distributions of the logarithms of individual particle effective diffusivities ($D_{eff}$) at a time scale of 1 s. Particles were prepared with the emulsification method using PLGA-PEG (6wt% PEG). Data represent three independent experiments with $\geq$ 120 nanoparticles tracked for each experiment. Error bars are presented as s.e.m.

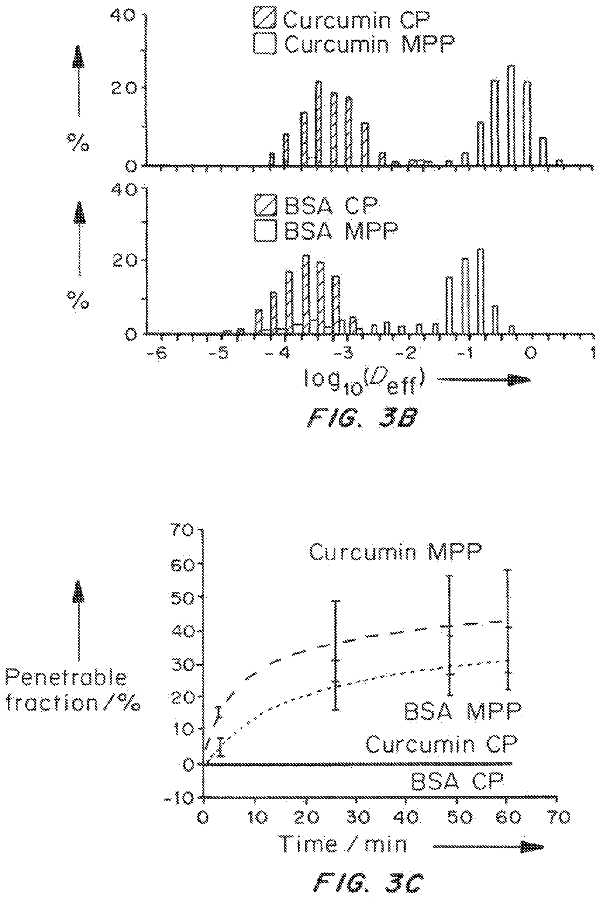

Figure 3a is a graph showing the ensemble-averaged geometric mean square displacement <MSD/ $\mu$m$^2$> as a function of time scale. Figure 3b is a graph showing the distributions of the logarithms of individual particle effective diffusivities ($D_{eff}$) at a time scale of 1 s. Figure 3c is a graph showing the estimated fraction of particles predicted to be capable of penetrating a 30 $\mu$m thick mucus layer over time. Data represent three independent experiments with $\geq$ 120 nanoparticles tracked for each experiment. Error bars are presented as s.e.m.

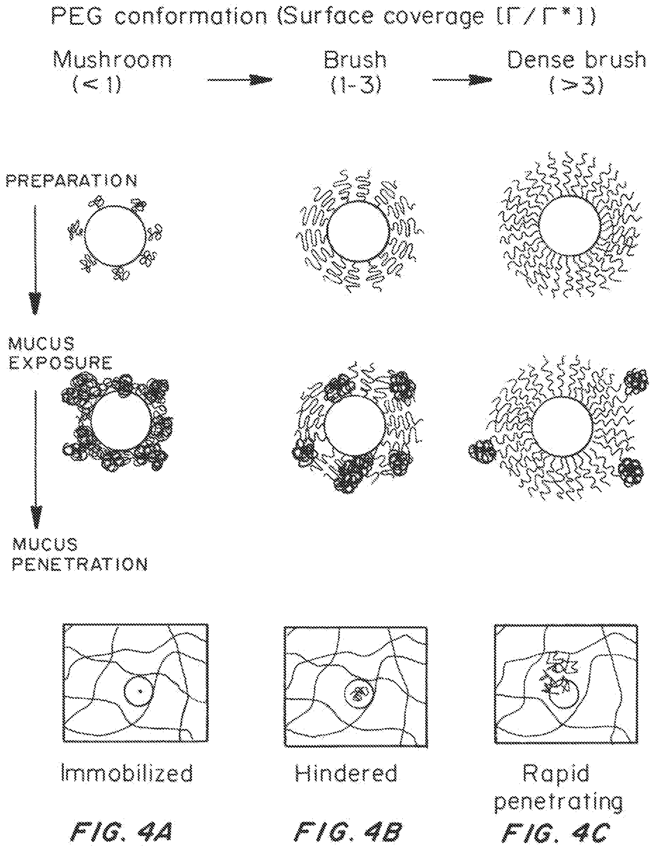

Figures 4a-c are schematics illustrating the influence of surface PEG coverage ([$\Gamma/\Gamma^*$] ) on mucus penetration of nanoparticles. Figures 4a-c shows the preparation of PLGA-PEG nanoparticles with surface PEG coating at increasing coverage. As surface PEG coverage increases, PEG regime changes from mushroom (neighboring PEG chains do not overlap, [$\Gamma/\Gamma^*$] <1, Figure 4a), to brush (neighboring PEG chains overlap, 1<[$\Gamma/\Gamma^*$]<3, Figure 4b), to dense brush ([$\Gamma/\Gamma^*$]>3, Figure 4c). At low PEG coverage ([$\Gamma/\Gamma^*$] <1, Figure 4a), mucin fibers strongly adhere to nanoparticle core. At middle PEG coverage (1<[Γ/Γ*]<3, Figure 4b), mucin fibers still can partially absorb to the nanoparticle core. At high ([Γ/Γ*]>3, Figure 4c) PEG coverage, the nanoparticle cores were completely shielded by the bioinert PEG corona resulting in no absorption of mucin to nanoparticles. Figure 4c shows that nanoparticles with low PEG coverage are immobilized in mucus, nanoparticles with middle PEG coverage are hindered or even immobilized in mucus, and nanoparticles with high and very high PEG coverage are able to rapidly penetrate mucus.

## DETAILED DESCRIPTION OF THE INVENTION

### I. Definitions

"Nanoparticle," as used herein, generally refers to a particle of any shape having a diameter from about 1 nm up to, but not including, about 1 micron, more preferably from about 5 nm to about 500 nm, most preferably from about 5 nm to about 100 nm. Nanoparticles having a spherical shape are generally referred to as "nanospheres".

"Mean particle size," as used herein, generally refers to the statistical mean particle size (diameter) of the particles in a population of particles. The diameter of an essentially spherical particle may be referred to as the physical or hydrodynamic diameter. The diameter of a non-spherical particle may refer preferentially to the hydrodynamic diameter. As used herein, the diameter of a non-spherical particle may refer to the largest linear distance between two points on the surface of the particle. Mean particle size can be measured using methods known in the art, such as dynamic light scattering.

"Mass Median Aerodynamic Diameter" (MMAD), as used herein, refers to the median aerodynamic size of a plurality of particles. The "aerodynamic diameter" is the diameter of a unit density sphere having the same settling velocity, generally in air, as a powder and is therefore a useful way to characterize an aerosolized powder or other dispersed particle or particle formulation in terms of its settling behavior. The aerodynamic diameter encompasses particle or particle shape, density, and physical size of the particle or particle. MMAD can be experimentally determined by methods known in the art, such as by cascade impaction.

"Tap Density," as used herein, refers to a measure of the density of a powder. Tap density can be determined using the method of USP Bulk Density and Tapped Density, United States Pharmacopia convention, Rockville, Md., 10th Supplement, 4950-4951, 1999. Features which can contribute to low tap density include irregular surface texture and porous structure.

"Monodisperse" and "homogeneous size distribution," are used interchangeably herein and describe a plurality of nanoparticles or microparticles where the particles have the same or nearly the same diameter or aerodynamic diameter. As used herein, a monodisperse distribution refers to particle distributions in which 80, 81, 82, 83, 84, 85, 86, 86, 88, 89, 90, 91, 92, 93, 94, 95% or greater of the distribution lies within 5% of the mass median diameter or aerodynamic diameter.

"Pulmonary administration," as used herein, refers to administration of a pharmaceutical formulation containing an active agent into the lungs by inhalation. As used herein, the term "inhalation" refers to intake of air to the alveoli. The intake of air can occur through the mouth or nose. The intake of air can occur by self-administration of a formulation while inhaling, or by administration via a respirator to a patient on a respirator.

"Pharmaceutically acceptable," as used herein, refers to compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio, in accordance with the guidelines of agencies such as the Food and Drug Administration.

"Biocompatible" and "biologically compatible," as used herein, generally refer to materials that are, along with any metabolites or degradation products thereof, generally non-toxic to the recipient, and do not cause any significant adverse effects to the recipient. Generally speaking, biocompatible materials are materials which do not elicit a significant inflammatory or immune response when administered to a patient.

"Molecular weight," as used herein, generally refers to the relative average chain length of the bulk polymer, unless otherwise specified. In practice, molecular weight can be estimated or characterized using various methods including gel permeation chromatography (GPC) or capillary viscometry. GPC molecular weights are reported as the weight-average molecular weight (Mw) as opposed to the number-average molecular weight (Mn). Capillary viscometry provides estimates of molecular weight as the inherent viscosity determined from a dilute polymer solution using a particular set of concentration, temperature, and solvent conditions.

"Hydrophilic," as used herein, refers to the property of having affinity for water. For example, hydrophilic polymers (or hydrophilic polymer segments) are polymers (or polymer segments) which are primarily soluble in aqueous solutions and/or have a tendency to absorb water. In general, the more hydrophilic a polymer is, the more that polymer tends to dissolve in, mix with, or be wetted by water.

"Hydrophobic," as used herein, refers to the property of lacking affinity for, or even repelling water. For example, the more hydrophobic a polymer (or polymer segment), the more that polymer (or polymer segment) tends to not dissolve in, not mix with, or not be wetted by water.

"Mucus," as used herein, refers to a viscoelastic natural substance containing primarily mucin glycoproteins and other materials, which protects epithelial surface of various organs/tissues, including respiratory, nasal, cervicovaginal, gastrointestinal, rectal, visual and auditory systems. "Sputum," as used herein, refers to highly viscoelastic mucus secretions consist of a variety of macromolecules such as DNA, actins and other cell debris released from dead cells in addition to mucin glycoproteins. "Sputum" is generally present in the pathogenic airways of patients afflicted by obstructive lung diseases, including but not limited to, asthma, COPD and CF. "CF mucus" and "CF sputum," as used herein, refer to mucus and sputum, respectively, from a patient suffering from cystic fibrosis.

"Mucus Degrading Agent," as used herein, refers to a substance which increases the rate of mucus clearance when administered to a patient. Mucus degrading agents are known in the art. *See,* for example, Hanes, J. et al. Gene Delivery to the Lung. in Pharmaceutical Inhalation Aerosol Technology, Marcel Dekker, Inc., New York: 489-539 (2003). Examples of mucus degrading agents include N-acetylcysteine (NAC), which cleaves disulfide and sulfhydryl bonds present in mucin. Other mucus degrading agents include mugwort, bromelain, papain, clerodendrum, acetylcysteine, bromhexine, carbocisteine, eprazinone, mesna, ambroxol, sobrerol, domiodol, denufosol, letosteine, stepronin, tiopronin, gelsolin, thymosin β4, neltenexine, erdosteine, and various DNases including rhDNase.

"CF Mucus-resistant/diffusive Particle," as used herein, refers to a particle which exhibits reduced or low mucoadhesion in CF mucus and which therefore passes through the CF mucus at a higher rate than other particles. Such particles may be characterized as having high diffusivity through CF mucus. In certain embodiments, CF mucus-resistant/diffusive particles possess an effective diffusivity in CF mucus of greater than about 0.01 $\mu m^2/s$, more preferably greater than about 0.5 $\mu m^2/s$, most preferably greater than about 1 $\mu m^2/s$. In preferred embodiments, a population of particles may be characterized as "CF mucus-resistant/diffusive" if at least 30%, more preferably at least 40%, most preferably at least 50% of the population of particles diffuse across a 10 $\mu m$ thick CF sputum layer within one hour.

"Cystic Fibrosis" (CF), as used herein, refers to an inherited genetic disease resulting from one or more mutations in the gene encoding the cystic fibrosis transmembrane conductance regulator (CFTR). In patients with cystic fibrosis, mutations in CFTR endogenously expressed in respiratory epithelia lead to reduced apical anion secretion causing an imbalance in ion and fluid transport. The resulting decrease in anion transport contributes to enhanced mucus accumulation in the lung and the accompanying microbial infections that ultimately cause death in CF patients. In addition to respiratory disease, CF patients typically suffer from gastrointestinal problems and pancreatic insufficiency that, if left untreated, result in death. Sequence analysis of the CFTR gene of CF chromosomes has revealed a variety of disease causing mutations. To date, more than 1000 disease causing mutations in the CF gene have been identified (http://www.genet.sickkids.on.ca/cftr/). The most prevalent mutation is a deletion of phenylalanine at position 508 of the CFTR amino acid sequence, and is commonly referred to as ΔF508-CFTR. This mutation occurs in approximately 70 percent of the cases of cystic fibrosis and is associated with a severe disease. Cystic fibrosis affects approximately one in every 2,500 infants in the United States.

"Cystic Fibrosis Transmembrane Conductance Regulator" (CFTR), as used herein, refers to a transmembrane protein critical for the maintenance of electrolyte transport throughout the body, including respiratory and digestive tissue. CFTR is composed of approximately 1480 amino acids that encode a protein made up of a tandem repeat of transmembrane domains, each containing six transmembrane helices and a nucleotide binding domain. The gene encoding CFTR has been identified and sequenced. *See* Gregory, R. J. et al. Nature 347:382386 (1990); Rich, D. P. et al. Nature 347:358-362 (1990), and Riordan, J. R. et al. Science 245:1066-1073 (1989).

"Nucleic Acid," as used herein, refers to DNA, RNA, and nucleic acid molecules modified to increase stability for a variety of therapeutic purposes. One example is a gene encoding the human cystic fibrosis transmembrane conductance regulator (CFTR) protein, analogs and variants thereof, that can be expressed in CF individuals to correct at least in part some of the symptoms characteristic of CF. This also include molecules such as DNA fragments including regions for introducing corrections or modifications into the gene, such as triple helix forming DNA, that can be used to correct the endogenous CF gene in at least some of the CF patient's genes. It should be noted that this term is not limited to CFTR genes but applied to every genetic material that can be used to treat, diagnose or cure disease.

The phrases "parenteral administration" and "administered parenterally" are art-recognized terms, and include modes of administration other than enteral and topical administration, such as injections, and include, but are not limited to, intravenous, intramuscular, intrapleural, intravascular, intrapericardial, intraarterial, intrathecal, intracapsular, intraorbital, intracardiac, intradennal, intraperitoneal, transtracheal, subcutaneous, subcuticular, intraarticular, subcapsular, subarachnoid, intraspinal, subconjunctivally, and intrastemal injection and infusion.

The term "surfactant" as used herein refers to an agent that lowers the surface tension of a liquid.

The term "therapeutic agent" refers to an agent that can be administered to prevent or treat a disease or disorder. Therapeutic agents can be a nucleic acid, a nucleic acid analog, a small molecule, a peptidomimetic, a protein, peptide, carbohydrate or sugar, lipid, or surfactant, or a combination thereof.

The term "treating" or preventing a disease, disorder or condition from occurring in an animal which may be predisposed to the disease, disorder and/or condition but has not yet been diagnosed as having it; inhibiting the disease, disorder or condition, e.g., impeding its progress; and relieving the disease, disorder, or condition, e.g., causing regression of the disease, disorder and/or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain. Any references in the description to treatments or methods of treatment refer to the compositions of the present invention for use in a method of treatment of the human (or animal) body by therapy.

The term "targeting moiety" as used herein refers to a moiety that localizes to or away from a specific locale. The moiety may be, for example, a protein, nucleic acid, nucleic acid analog, carbohydrate, or small molecule. The entity may be, for example, a therapeutic compound such as a small molecule, or a diagnostic entity such as a detectable label. The locale may be a tissue, a particular cell type, or a subcellular compartment. In one embodiment, the targeting moiety directs the localization of an active entity. The active entity may be a small molecule, protein, polymer, or metal. The active entity may be useful for therapeutic, prophylactic, or diagnostic purposes.

The term "therapeutically effective amount" refers to an amount of the therapeutic agent that, when incorporated into and/or onto particles described herein, produces some desired effect at a reasonable benefit/risk ratio applicable to any medical treatment. The effective amount may vary depending on such factors as the disease or condition being treated, the particular targeted constructs being administered, the size of the subject, or the severity of the disease or condition. One of ordinary skill in the art may empirically determine the effective amount of a particular compound without necessitating undue experimentation.

The terms "incorporated" and "encapsulated" refers to incorporating, formulating, or otherwise including an active agent into and/or onto a composition that allows for release, such as sustained release, of such agent in the desired application. The terms contemplate any manner by which a therapeutic agent or other material is incorporated into a polymer matrix, including, for example: attached to a monomer of such polymer (by covalent, ionic, or other binding interaction), physical admixture, enveloping the agent in a coating layer of polymer, incorporated into the polymer, distributed throughout the polymeric matrix, appended to the surface of the polymeric matrix (by covalent or other binding interactions), encapsulated inside the polymeric matrix, etc. The term "co-incorporation" or "co-encapsulation" refers to the incorporation of a therapeutic agent or other material and at least one other therapeutic agent or other material in a subject composition.

## II. Mucus-penetrating nanoparticles (MPPs)

### A. Core Polymer

Any number of biocompatible polymers can be used to prepare the nanoparticles. In one embodiment, the biocompatible polymer(s) is biodegradable. In another embodiment, the particles are non-degradable. In other embodiments, the particles are a mixture of degradable and non-degradable particles.

Exemplary polymers include, but are not limited to, cyclodextrin-containing polymers, in particular cationic cyclodextrin-containing polymers, such as those described in U.S. Patent No. 6,509,323,

polymers prepared from lactones, such as poly(caprolactone) (PCL),

polyhydroxy acids and copolymers thereof such as poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-co-D,L-lactide), and blends thereof,

polyalkyl cyanoacralate,

polyurethanes,

polyamino acids such as poly-L-lysine (PLL), poly(valeric acid), and poly-L-glutamic acid,

hydroxypropyl methacrylate (HPMA),

polyanhydrides,

polyesters,

polyorthoesters,

poly(ester amides),

polyamides,

poly(ester ethers),

polycarbonates,

polyalkylenes such as polyethylene and polypropylene, polyalkylene glycols such as poly(ethylene glycol) (PEG), polyalkylene oxides (PEO),

polyalkylene terephthalates such as poly(ethylene terephthalate), ethylene vinyl acetate polymer (EVA),

polyvinyl alcohols (PVA),

polyvinyl ethers,
polyvinyl esters such as poly(vinyl acetate),
polyvinyl halides such as poly(vinyl chloride) (PVC), polyvinylpyrrolidone, polysiloxanes,
polystyrene (PS),
celluloses including derivatized celluloses such as alkyl celluloses, hydroxyalkyl celluloses, cellulose ethers, cellulose esters, nitro celluloses, hydroxypropylcellulose, and carboxymethylcellulose,
polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(lauryl(meth)acrylate), poly(phenyl(meth)acrylate), poly(methyl acrylate), poly(isopropyl acrylate), poly(isobutyl acrylate), poly(octadecyl acrylate) (jointly referred to herein as "polyacrylic acids"),
polydioxanone and its copolymers,
polyhydroxyalkanoates,
polypropylene fumarate,
polyoxymethylene,
poloxamers,
poly(butyric acid),
trimethylene carbonate, and
polyphosphazenes,.
Copolymers of the above, such as random, block, or graft copolymers, or blends of the polymers listed above can also be used.

Functional groups on the polymer can be capped to alter the properties of the polymer and/or modify (e.g., decrease or increase) the reactivity of the functional group. For example, the carboxyl termini of carboxylic acid contain polymers, such as lactide- and glycolide-containing polymers, may optionally be capped, e.g., by esterification, and the hydroxyl termini may optionally be capped, e.g. by etherification or esterification.

Copolymers of PEG or derivatives thereof with any of the polymers described above may be used to make the polymeric particles. In certain embodiments, the PEG or derivatives may be located in the interior positions of the copolymer. Alternatively, the PEG or derivatives may locate near or at the terminal positions of the copolymer. For example, one or more of the polymers above can be terminated with a block of polyethylene glycol. In some embodiments, the core polymer is a blend of pegylated polymer and non-pegylated polymer, wherein the base polymer is the same (e.g., PLGA and PLGA-PEG) or different (e.g., PLGA-PEG and PLA). In certain embodiments, the nanoparticles are formed under conditions that allow regions of PEG to phase separate or otherwise locate to the surface of the particles. The surface-localized PEG regions alone may perform the function of, or include, the surface-altering agent. In particular embodiments, the particles are prepared from one or more polymers terminated with blocks of polyethylene glycol as the surface-altering material.

The weight average molecular weight can vary for a given polymer but is generally from about 1000 Daltons to 1,000,000 Daltons, 1000 Daltons to 500,000 Dalton, 1000 Daltons to 250,000 Daltons, 1000 Daltons to 100,000 Daltons, 5,000 Daltons to 100,000 Daltons, 5,000 Daltons to 75,000 Daltons, 5,000 Daltons to 50,000 Daltons, or 5,000 Daltons to 25,000 Daltons.

Examples of preferred natural polymers include proteins such as albumin, collagen, gelatin and prolamines, for example, zein, and polysaccharides such as alginate.

In some embodiments, the particles may be used as nanoparticle gene carriers. In these embodiments, the particles can be formed of one or more polycationic polymers which complex with one or more nucleic acids which are negatively charged.

The cationic polymer can be any synthetic or natural polymer bearing at least two positive charges per molecule and having sufficient charge density and molecular size to bind to nucleic acid under physiological conditions (*i.e.*, pH and salt conditions encountered within the body or within cells). In certain embodiments, the polycationic polymer contains one or more amine residues.

Suitable cationic polymers include, for example, polyethylene imine (PEI), polyallylamine, polyvinylamine, polyvinylpyridine, aminoacetalized poly(vinyl alcohol), acrylic or methacrylic polymers (for example, poly(N,N-dimethylaminoethylmethacrylate)) bearing one or more amine residues, polyamino acids such as polyornithine, polyarginine, and polylysine, protamine, cationic polysaccharides such as chitosan, DEAE-cellulose, and DEAE-dextran, and polyamidoamine dendrimers (cationic dendrimer), as well as copolymers and blends thereof. In preferred embodiments, the polycationic polymer is PEI.

Cationic polymers can be either linear or branched, can be either homopolymers or copolymers, and when containing amino acids can have either L or D configuration, and can have any mixture of these features. Preferably, the cationic polymer molecule is sufficiently flexible to allow it to form a compact complex with one or more nucleic acid molecules.

In some embodiments, the polycationic polymer has a molecular weight of between about 5,000 Daltons and about 100,000 Daltons, more preferably between about 5,000 and about 50,000 Daltons, most preferably between about 10,000 and about 35,000 Daltons.

## B. Materials that promote diffusion through mucus

The nanoparticles of the present invention are formed by emulsion of one or more core polymers, one or more surface altering materials, and one or more low molecular weight emulsifiers having a molecular weight less than 1500 amu, wherein the one or more surface altering materials are selected from the group consisting of polyethylene glycol (PEG) and poloxamer, or wherein the particles are formed from block copolymers containing PEG.

More generally, however, in accordance with the disclosure of the present application, the nanoparticles preferably are coated with or contain one or more surface altering agents or materials. "Surface-altering agents", as used herein refers to an agent or material which modifies one or more properties of the particles for the surface, including, but not limited to, hydrophilicity (e.g., makes the particles more or less hydrophilic), surface charge (e.g., makes the surface neutral or near neutral or more negative or positive), and/or enhances transport in or through bodily fluids and/or tissues, such as mucus. In some embodiments, the surface-altering material provides a direct therapeutic effect, such as reducing inflammation.

Examples of the surface-altering agents disclosed herein include proteins, including anionic proteins (e.g., albumin), surfactants, sugars or sugar derivatives (e.g., cyclodextrin), therapeutics agents, and polymers such as heparin; these surface-altering agents are outside the scope of the claims. The surface altering agents according to the invention are selected from polyethylene glycol ("PEG") and poloxamers (polyethylene oxide block copolymers). The most preferred material is PEG.

Examples of surfactants include, but are not limited to, L-$\alpha$-phosphatidylcholine (PC), 1,2-dipalmitoylphosphatidycholine (DPPC), oleic acid, sorbitan trioleate, sorbitan mono-oleate, sorbitan monolaurate, polyoxyethylene (20) sorbitan monolaurate, polyoxyethylene (20) sorbitan monooleate, natural lecithin, oleyl polyoxyethylene (2) ether, stearyl polyoxyethylene (2) ether, lauryl polyoxyethylene (4) ether, block copolymers of oxyethylene and oxypropylene, synthetic lecithin, diethylene glycol dioleate, tetrahydrofurfuryl oleate, ethyl oleate, isopropyl myristate, glyceryl monooleate, glyceryl monostearate, glyceryl monoricinoleate, cetyl alcohol, stearyl alcohol, polyethylene glycol 400, cetyl pyridinium chloride, benzalkonium chloride, olive oil, glyceryl monolaurate, corn oil, cotton seed oil, and sunflower seed oil, lecithin, oleic acid, and sorbitan trioleate.

In one embodiment of the present invention, the nanoparticles are coated with or contain polyethylene glycol (PEG). PEG can be applied as coating onto the surface of the particles. Alternatively, the PEG can be in the form of blocks covalently bound (e.g., in the interior or at one or both terminals) to the core polymer used to form the particles. In particular embodiments, the particles are formed from block copolymers containing PEG. In more particular embodiments, the particles are prepared from block copolymers containing PEG, wherein PEG is covalently bound to the terminal of the base polymer.

Representative PEG molecular weights include 300 Da, 600 Da, 1 kDa, 2 kDa, 3 kDa, 4 kDa, 6 kDa, 8 kDa, 10 kDa, 15 kDa, 20 kDa, 30 kDa, 50 kDa, 100 kDa, 200 kDa, 500 kDa, and 1 MDa and all values within the range of 300 Daltons to 1 MDa. In preferred embodiments, the PEG has a molecular weight of about 5kD. PEG of any given molecular weight may vary in other characteristics such as length, density, and branching.

## i. Evaluating surface density

Surface density of poly(ethylene glycol) (PEG) on nanoparticles is a key parameter in determining their successful applications *in-vivo.* The controlled delivery of drugs to mucosal surfaces is challenging because of the presence of the protective mucus layer, and the mucus-penetrating particles show promise at improved drug distribution, retention and efficacy at mucosal surfaces. The dense coating of PEG on biodegradable nanoparticles can allow rapid penetration through mucus because of the greatly reduced adhesive interaction between mucus constituents and nanoparticles. However, it still remains unclear how to optimally control the surface PEG density and to prepare biodegradable mucus-penetrating nanoparticles for in-vivo application.

Different methods have been employed to assess the surface PEG density on nanoparticles, including those that directly measure changes to physiochemical properties of nanoparticles, such as surface charge and hydrodynamic diameter. However, these methods cannot provide quantitative information about the number of PEG chains per $nm^2$ of the particle surface.

In order to directly quantify the surface PEG density, various techniques have been applied. Thermogravimetric analysis (TGA) can be used to calculate PEG content, but it is limited to inorganic materials and also requires the use of relatively large quantity of samples.

The reactions of dye and reagents (such as fluorescence dye) to functional PEG were widely used for PEG quantification. In these methods, the un-reacted PEG molecules with functional groups (such as -SH, -NH$_2$, etc) were

quantified by fluorescent assay or colorimetric quantification after the reaction with certain reagents, and the surface PEG density was achieved by subtracting the un-reacted PEG portion in supernatant. However, these methods are limited to surface PEGylation and functional PEG. Similar methods used to quantify surface PEG density on PRINT nanoparticles by the measurement of signal of un-reacted fluoresein-PEG in supernatant are limited to surface modification of nanoparticles with PEG. Thus these quantitative assays are not suitable for determining the PEG density on biodegradable nanoparticles prepared from PEG-containing block copolymers, such as the widely used poly(lactic-co-glycolic acid)-poly(ethylene glycol) (PLGA-PEG) and poly(lactic acid)-poly(ethylene glycol) (PLA-PEG).

Nuclear magnetic resonance (NMR) can be used to assess the surface PEG density on PEG-containing polymeric nanoparticles described herein, both qualitatively and quantitatively (PEG peak typically observed ~3.65 ppm). When nanoparticles are dispersed within the NMR solvent $D_2O$, only the surface PEG, not the PEG embedded within the core, can be directly detected by NMR. Therefore, NMR provides a means for directly measure the surface density of PEG.

In some embodiments, PEG surface density can be controlled by preparing the particles from a mixture of pegylated and non-pegylated particles. For example, the surface density of PEG on PLGA nanoparticles can be precisely controlled by preparing particles from a mixture of poly(lactic-co-glycolic acid) and poly(ethylene glycol) (PLGA-PEG). Quantitative $^1H$ nuclear magnetic resonance (NMR) can be used to measure the surface PEG density on nanoparticles. Multiple particle tracking in human mucus and the study of mucin binding and tissue distribution in mouse vagina revealed that there exists a PEG density threshold, which is approximately, 10-16 PEG chains/100nm$^2$, for PLGA-PEG nanoparticles to be effective in penetrating mucus. This density threshold may vary depending on a variety of factors including the core polymer used to prepare the particles, particle size, and/or molecular weight of PEG.

The density of the coating can be varied based on a variety of factors including the surface altering material and the composition of the particle. In one embodiment, the density of the surface altering material, such as PEG, as measured by $^1H$ NMR is at least, 0.1, 0.2, 0.5, 0.8, 1, 2, 5, 8, 10, 15, 20, 25, 40, 50, 60, 75, 80, 90, or 100 chains per nm$^2$. The range above is inclusive of all values from 0.1 to 100 units per nm$^2$.

In particular embodiments, the density of the surface altering material, such as PEG, is from about 1 to about 25 chains/nm$^2$, from about 1 to about 20 chains/nm$^2$, from about 5 to about 20 chains/nm$^2$, from about 5 to about 18 chains/nm$^2$, from about 5 to about 15 chains/nm$^2$, or from about 10 to about 15 chains/nm$^2$. The concentration of the surface altering material, such as PEG, can also be varied. In some embodiments, the target concentration of the surface altering material, such as PEG, is at least 0.5, 1, 2, 3, 4, 5, 6, 7, 8,9, 10, 11, 12, 13, 14, 15, 16, 17, 18, 19, 20, 21,22, 23, 24, or 25% or higher. The range above is inclusive of all values from 0.5% to 25%. In another embodiment, the concentration of the surface altering material, such as PEG, in the particle is at least 0.5, 1, 2, 3, 4, 5, 6, 7, 8, 9, 10, 11, 12, 13, 14, 15, 16, 17, 18, 19, 20, 21, 22, 23, 24, or 25%. The range above is inclusive of all values from 0.5% to 25%. In still other embodiments, the surface altering material content (e.g., PEG) on the surface of the particles is at least 0.5, 1, 2, 3, 4, 5, 6, 7, 8, 9, 10, 11, 12, 13, 14, 15, 16, 17, 18, 19, 20, 21, 22, 23, 24, or 25%. The range above is inclusive of all values from 0.5% to 25%.

In particular embodiments, the density of the surface-altering material (e.g., PEG) is such that the that the surface-altering material (e.g. PEG) adopted an extended brush configuration.

In other embodiments, the mass of the surface-altering moiety is at least 1/10,000, 1/7500, 1/5000, 1/4000, 1/3400, 1/2500, 1/2000, 1/1500, 1/1000, 1/750, 1/500, 1/250, 1/200, 1/150, 1/100, 1/75, 1/50, 1/25, 1/20, 1/5, 1/2, or 9/10 of the mass of the particle. The range above is inclusive of all vales from 1/10,000 to 9/10.

## C. Emulsifier

The nanoparticles of the present invention contain a low molecular weight emulsifier having a molecular weight less than 1500 amu. The emulsifier is incorporated into the particle during particle formation and therefore is a component of the finished particle. The emulsifier can be encapsulated within the particle, be dispersed in whole or in part within the polymer matrix (e.g., part of the emulsifier extends out from the polymer matrix), and/or is associated (e.g., covalently or non-covalently) with the surface of the particle.

"Low molecular weight", as used herein, generally refers to an emulsifier having a molecular weight less than 1500, 1400, 1300, 1200, 1100, 1000, 900, 800, 700, 600, 500, 400, or 300 amu. In some embodiments, the molecular weight is less than 1300 amu. In some embodiments, the molecular weight is from about 300 amu to about 1200 amu.

The emulsifier can be positively charged, negatively charged, or neutral. Examples of negatively charged emulsifiers include, but are not limited to, cholic acid sodium salt (CHA, MW = 430) and dioctyl sulfosuccinate sodium (DSS, MW = 455). Examples of positively charged emulsifiers include, but are not limited to, hexadecyltrimethyl ammonium bromide (CTAB, MW = 364). Examples of neutral emulsifiers include, but are not limited to, saponin (MW = 1191), TWEEN 20 (MW = 1,225), TWEEN 80 (MW = 1310), and sugar ester D1216 (sucrose laurate, SE, MW = 524).

In addition to having a low molecular weight, the emulsifier must be capable of suitably stabilizing the emulsion droplets during particle formation in order to prevent particle aggregation. The emulsification capability of a particular emulsifier can be calculated using the equation below and is expressed as a percent.

$$\text{Emulsification capability} = \text{weight of nanoparticles}/(\text{weight of nanoparticles} + \text{weight of aggregated particles}) \times 100\%$$

In some embodiments, the emulsification capability is at least 50, 55, 60, 65, 70, 75, 80, 85, 86, 87, 88, 89, 90, 91, 92, 93, 94, or 95%. This range is inclusive of all values between 50 and 95.

In addition to suitably stabilizing the emulsion droplets to prevent aggregate formation, the stabilizer must be small enough to be completely shielded at the particle surface by the surface altering material corona (e.g., PEG) to provide a neutral or near neutral surface charge. The transport of charged particles may be hindered due to the interaction of the charged particles with oppositely charged species *in vivo.* For example, the ability of the particles to penetrate mucus rapidly is dependent, at least in part, on the surface charge of the particles. Therefore, the emulsifier(s) must be small enough that the emulsifier, if charged (e.g., positively or negatively), the charge is shielded by the corona of the surface altering material (e.g., PEG) such that the surface charge is zero or essentially zero, e.g., -10 to 10 ev, -5 to 5 ev, -3 to 3 ev, -2 to 2 ev, or -1 to 1 ev.

### D. Therapeutic, prophylactic, nutraceutical and/or diagnostic agent

### 1. Therapeutic agents

In some embodiments, the nanoparticles of the present invention have encapsulated therein, dispersed therein, and/or covalently or non-covalently associate with the surface one or more therapeutic agents. The therapeutic agent can be a small molecule, protein, polysaccharide or saccharide, nucleic acid molecule and/or lipid.

### i. Small molecule therapeutic agents

Exemplary classes of small molecule therapeutic agents include, but are not limited to, analgesics, anti-inflammatory drugs, antipyretics, antidepressants, antiepileptics, antiopsychotic agents, neuroprotective agents, anti-proliferatives, such as anti-cancer agent, anti-infectious agents, such as antibacterial agents and antifungal agents, antihistamines, antimigraine drugs, antimuscarinics, anxioltyics, sedatives, hypnotics, antipsychotics, bronchodilators, anti-asthma drugs, cardiovascular drugs, corticosteroids, dopaminergics, electrolytes, gastro-intestinal drugs, muscle relaxants, nutritional agents, vitamins, parasympathomimetics, stimulants, anorectics and anti-narcoleptics.

### ii. Nucleic acids

In some embodiments, the agent is one or more nucleic acids. The nucleic acid can alter, correct, or replace an endogenous nucleic acid sequence. The nucleic acid is used to treat cancers, correct defects in genes in other pulmonary diseases and metabolic diseases affecting lung function, genes such as those for the treatment of Parkinsons and ALS where the genes reach the brain through nasal delivery.

Gene therapy is a technique for correcting defective genes responsible for disease development. Researchers may use one of several approaches for correcting faulty genes:

- A normal gene may be inserted into a nonspecific location within the genome to replace a nonfunctional gene. This approach is most common.
- An abnormal gene could be swapped for a normal gene through homologous recombination.
- The abnormal gene could be repaired through selective reverse mutation, which returns the gene to its normal function.
- The regulation (the degree to which a gene is turned on or off) of a particular gene could be altered.

The nucleic acid can be a DNA, RNA, a chemically modified nucleic acid, or combinations thereof. For example, methods for increasing stability of nucleic acid half-life and resistance to enzymatic cleavage are known in the art, and can include one or more modifications or substitutions to the nucleobases, sugars, or linkages of the polynucleotide. The nucleic acid can be custom synthesized to contain properties that are tailored to fit a desired use. Common modifications include, but are not limited to use of locked nucleic acids (LNAs), unlocked nucleic acids (UNAs), morpholinos, peptide nucleic acids (PNA), phosphorothioate linkages, phosphonoacetate linkages, propyne analogs, 2'-O-methyl RNA, 5-Me-dC, 2-5' linked phosphodiester linage, Chimeric Linkages (Mixed phosphorothioate and phosphodiester linkages and modifications), conjugation with lipid and peptides, and combinations thereof.

In some embodiments, the nucleic acid includes internucleotide linkage modifications such as phosphate analogs having achiral and uncharged intersubunit linkages (e.g., Sterchak, E. P. et al., Organic Chem., 52:4202, (1987)), or uncharged morpholino-based polymers having achiral intersubunit linkages (see, e.g., U.S. Patent No. 5,034,506). Some internucleotide linkage analogs include morpholidate, acetal, and polyamide-linked heterocycles. Other backbone and linkage modifications include, but are not limited to, phosphorothioates, peptide nucleic acids, tricyclo-DNA, decoy oligonucleotide, ribozymes, spiegelmers (containing L nucleic acids, an apatamer with high binding affinity), or CpG oligomers.

Phosphorothioates (or S-oligos) are a variant of normal DNA in which one of the nonbridging oxygens is replaced by a sulfur. The sulfurization of the internucleotide bond dramatically reduces the action of endo-and exonucleases including 5' to 3' and 3' to 5' DNA POL 1 exonuclease, nucleases S1 and P1, RNases, serum nucleases and snake venom phosphodiesterase. In addition, the potential for crossing the lipid bilayer increases. Because of these important improvements, phosphorothioates have found increasing application in cell regulation. Phosphorothioates are made by two principal routes: by the action of a solution of elemental sulfur in carbon disulfide on a hydrogen phosphonate, or by the more recent method of sulfurizing phosphite triesters with either tetraethylthiuram disulfide (TETD) or 3H-1, 2-bensodithiol-3-one 1, 1-dioxide (BDTD).4 The latter methods avoid the problem of elemental sulfur's insolubility in most organic solvents and the toxicity of carbon disulfide. The TETD and BDTD methods also yield higher purity phosphorothioates.

Peptide nucleic acids (PNA) are molecules in which the phosphate backbone of oligonucleotides is replaced in its entirety by repeating N-(2-aminoethyl)-glycine units and phosphodiester bonds are replaced by peptide bonds. The various heterocyclic bases are linked to the backbone by methylene carbonyl bonds. PNAs maintain spacing of heterocyclic bases that is similar to oligonucleotides, but are achiral and neutrally charged molecules. Peptide nucleic acids are typically comprised of peptide nucleic acid monomers. The heterocyclic bases can be any of the standard bases (uracil, thymine, cytosine, adenine and guanine) or any of the modified heterocyclic bases described below. A PNA can also have one or more peptide or amino acid variations and modifications. Thus, the backbone constituents of PNAs may be peptide linkages, or alternatively, they may be non-peptide linkages. Examples include acetyl caps, amino spacers such as 8-amino-3,6-dioxaoctanoic acid (referred to herein as O-linkers), and the like. Methods for the chemical assembly of PNAs are well known.

In some embodiments, the nucleic acid includes one or more chemically-modified heterocyclic bases including, but are not limited to, inosine, 5-(1-propynyl) uracil (pU), 5-(1-propynyl) cytosine (pC), 5-methylcytosine, 8-oxo-adenine, pseudocytosine, pseudoisocytosine, 5 and 2-amino-5-(2'-deoxy-$\beta$-D-ribofuranosyl)pyridine (2-aminopyridine), and various pyrrolo- and pyrazolopyrimidine derivatives, 4-acetylcytosine, 8-hydroxy-N-6-methyladenosine, aziridinylcytosine, 5-(carboxyhydroxylmethyl) uracil, 5-bromouracil, 5-carboxymethylaminomethyl-2-thiouracil, 5-carboxymethylaminomethyluracil, dihydrouracil, N6-isopentenyladenine, 1-methyladenine, 1-methylpseudouracil, 1-methyl guanine, 1-methylinosine, 2,2-dimethylguanine, 2-methyladenine, 2-methylguanine, 3-methylcytosine , N6-methyladenine, 7-methylguanine, 5-methylaminomethyluracil, 5-methoxy-aminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, 2,6-diaminopurine, and 2'-modified analogs such as, but not limited to O-methyl, amino-, and fluoro-modified analogs. Inhibitory RNAs modified with 2'-flouro (2'-F) pyrimidines appear to have favorable properties in vitro. Moreover, one report recently suggested 2'-F modified siRNAs have enhanced activity in cell culture as compared to 2'-OH containing siRNAs. 2'-F modified siRNAs are functional in mice but that they do not necessarily have enhanced intracellular activity over 2'-OH siRNAs.

In some embodiments the nucleic acid includes one or more sugar moiety modifications, including, but are not limited to, 2'-O-aminoethoxy, 2'-O-amonioethyl (2'-OAE), 2'-O-methoxy, 2'-O-methyl, 2-guanidoethyl (2'-OGE), 2'-O,4'-C-methylene (LNA), 2'-O-(methoxyethyl) (2'-OME) and 2'-O-(N-(methyl)acetamido) (2'-OMA).

Methods of gene therapy typically rely on the introduction into the cell of a nucleic acid molecule that alters the genotype of the cell. Introduction of the nucleic acid molecule can correct, replace, or otherwise alters the endogenous gene via genetic recombination. Methods can include introduction of an entire replacement copy of a defective gene, a heterologous gene, or a small nucleic acid molecule such as an oligonucleotide. For example, corrective gene can be introduced into a nonspecific location within the host's genome. This approach typically requires delivery systems to introduce the replacement gene into the cell, such as genetically engineered viral vectors.

Methods to construct expression vectors containing genetic sequences and appropriate transcriptional and translational control elements are well known in the art. These methods include in vitro recombinant DNA techniques, synthetic techniques, and in vivo genetic recombination. Expression vectors generally contain regulatory sequences necessary elements for the translation and/or transcription of the inserted coding sequence. For example, the coding sequence is preferably operably linked to a promoter and/or enhancer to help control the expression of the desired gene product. Promoters used in biotechnology are of different types according to the intended type of control of gene expression. They can be generally divided into constitutive promoters, tissue-specific or development-stage-specific promoters, inducible promoters, and synthetic promoters.

Viral vectors include adenovirus, adeno-associated virus, herpes virus, vaccinia virus, polio virus, AIDS virus, neuronal trophic virus, Sindbis and other RNA viruses, including these viruses with the HIV backbone.

Also useful are any viral families which share the properties of these viruses which make them suitable for use as vectors. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA.

Gene targeting via target recombination, such as homologous recombination (HR), is another strategy for gene correction. Gene correction at a target locus can be mediated by donor DNA fragments homologous to the target gene (Hu, et al., Mol. Biotech., 29:197-210 (2005); Olsen, et al., J. Gene Med., 7:1534-1544 (2005)). One method of targeted recombination includes the use of triplex-forming oligonucleotides (TFOs) which bind as third strands to homopurine/homopyrimidine sites in duplex DNA in a sequence-specific manner. Triplex forming oligonucleotides can interact with either double-stranded or single-stranded nucleic acids. When triplex molecules interact with a target region, a structure called a triplex is formed, in which there are three strands of DNA forming a complex dependent on both Watson-Crick and Hoogsteen base-pairing. Triplex molecules are preferred because they can bind target regions with high affinity and specificity. It is preferred that the triplex forming molecules bind the target molecule with a Kd less than $10^{-6}$, $10^{-8}$, $10^{-10}$, or $10^{-12}$.

Methods for targeted gene therapy using triplex-forming oligonucleotides (TFO's) and peptide nucleic acids (PNAs) are described in U.S. Published Application No. 20070219122 and their use for treating infectious diseases such as HIV are described in U.S. Published Application No. 2008050920. The triplex-forming molecules can also be tail clamp peptide nucleic acids (tcPNAs), such as those described in U.S. Published Application No.2011/0262406. Highly stable PNA:DNA:PNA triplex structures can be formed from strand invasion of a duplex DNA with two PNA strands. In this complex, the PNA/DNA/PNA triple helix portion and the PNA/DNA duplex portion both produce displacement of the pyrimidine-rich triple helix, creating an altered structure that has been shown to strongly provoke the nucleotide excision repair pathway and to activate the site for recombination with the donor oligonucleotide. Two PNA strands can also be linked together to form a bis-PNA molecule. The triplex-forming molecules are useful to induce site-specific homologous recombination in mammalian cells when used in combination with one or more donor oligonucleotides which provides the corrected sequence. Donor oligonucleotides can be tethered to triplex-forming molecules or can be separate from the triplex-forming molecules. The donor oligonucleotides can contain at least one nucleotide mutation, insertion or deletion relative to the target duplex DNA.

Double duplex-forming molecules, such as a pair of pseudocomplementary oligonucleotides, can also induce recombination with a donor oligonucleotide at a chromosomal site. Use of pseudocomplementary oligonucleotides in targeted gene therapy is described in U.S. Published Application No. 2011/0262406. Pseudocomplementary oligonucleotides are complementary oligonucleotides that contain one or more modifications such that they do not recognize or hybridize to each other, for example due to steric hindrance, but each can recognize and hybridize to complementary nucleic acid strands at the target site. In some embodiments, pseudocomplementary oligonucleotides are pseudocomplemenary peptide nucleic acids (pcPNAs). Pseudocomplementary oligonucleotides can be more efficient and provide increased flexibility over methods of induced recombination such as triple-helix oligonucleotides and bis-peptide nucleic acids which require a polypurine sequence in the target double-stranded DNA.

## 2. Diagnostic Agents

Exemplary diagnostic materials include paramagnetic molecules, fluorescent compounds, magnetic molecules, and radionuclides. Suitable diagnostic agents include, but are not limited to, x-ray imaging agents and contrast media. Radionuclides also can be used as imaging agents. Examples of other suitable contrast agents include gases or gas emitting compounds, which are radioopaque. Nanoparticles can further include agents useful for determining the location of administered particles. Agents useful for this purpose include fluorescent tags, radionuclides and contrast agents.

For those embodiments where the one or more therapeutic, prophylactic, and/or diagnostic agents are encapsulated within a polymeric nanoparticle and/or associated with the surface of the nanoparticle, the percent drug loading is from about 1% to about 80%, from about 1% to about 50%, preferably from about 1% to about 40% by weight, more preferably from about 1% to about 20% by weight, most preferably from about 1% to about 10% by weight. The ranges above are inclusive of all values from 1% to 80%. For those embodiments where the agent is associated with the surface of the particle, the percent loading may be higher since the amount of drug is not limited by the methods of encapsulation. In some embodiments, the agent to be delivered may be encapsulated within a nanoparticle and associated with the surface of the particle. Nutraceuticals can also be incorporated. These may be vitamins, supplements such as calcium or biotin, or natural ingredients such as plant extracts or phytohormones.

## E. Properties of the particles

### 1. Surface charge and particle size

In order to facilitate their diffusion through mucus, the nanoparticles of the present invention possess a near neutral surface charge of between 10 mV and -10 mV when dispersed in 10 mM NaCl solution at pH 7. In certain embodiments, the nanoparticle possess a $\zeta$-potential of between about 5 mV and about -5 mV, preferably between about 3 mV and about -3 mV, more preferably between about 2 mV and about -2 mV. As discussed above, the particles described herein contain one or more low molecular weight emulsifiers. The emulsifier can be neutral, in which case the emulsifier has little or no effect on the surface charge of the particle. However, in some case, the emulsifier positively or negatively charged. In these embodiments, the surface altering material (e.g., PEG) must be present in sufficient density to form a corona which shields the positively or negatively charged emulsifier resulting in an effectively neutral surface.

While the particles described herein are referred to nanoparticles, and thus typically have an average diameter in the range of 1 nm up to, but not including, about 1 micron, more preferably from about 5 nm to about 500 nm, most preferably from about 5 nm to about 100 nm. In certain embodiments, the average diameter of the particles is form about 100 nm to about 150 nm. The conditions and/or materials used to prepare the particles can be varied to vary the size of the particles.

In certain embodiments, the nanoparticles retain their particle size and $\zeta$-potential after nebulization or storage for at least 1 month, more preferably at least 2 months, most preferably at least 3 months at 4°C.

### 2. Effect of emulsifier on transport ability

In some embodiments, the particles are administered to penetrate to the mucus for drug delivery to the mucosa. The particles described herein contain a surface-altering material which can enhance transport through the mucus. For example, PEG-containing block copolymers can self-assemble to form dense, muco-inert PEG coatings on the surface of emulsion droplets formed by the emulsification method, but only if low molecular weight (MW) emulsifiers are used in place of conventional higher weight or high weight emulsifiers, such as PVA. The low MW emulsifiers produced, on average, a several-thousand-fold increase in the mean-square-displacement (<MSD>) of nanoparticles in CVM, at a time scale of 1s, relative to nanoparticles prepared with high MW emulsifiers.

Furthermore, the nanoparticles of the present invention can penetrate CVM with effective speeds less than 25-fold, more preferably less than 10-fold, slower than the same particles in water. For example, the PEG-containing diblock copolymers, poly(lactic-acid)-b-PEG5k (PLA-PEG5k, Mn ~95kDa) and poly($\varepsilon$-caprolactone)-b-PEG5k (PCL-PEG5k, Mn ~78kDa) were also evaluated. Nanoparticles prepared from these two polymers and PVA were immobilized in CVM while rapid mucus penetration was observed for nanoparticles made using a low MW emulsifier, CHA, with effective diffusivities similar to those measured for PLGA-PEG5k nanoparticles.

In some embodiments, the particles described herein (prepared with low molecular weight emulsifier) exhibited transport rates of at least 500, 1000, 1500, 2000, 2500, 3000, 3500, 4000, 4500, 5000, 5500, 6000, 7000, 8000, 9000, or 10000 times greater than the particles prepared with PVA and/or exhibited effectives speeds less than 20, 19, 18, 17, 16, 15, 14, 13, 12, 11, 10, 9, 8, 7, 6, 5, 4 or 3-fold slower than the same particles in water.

In the absence of PEG, PLGA/CHA nanoparticles had a highly anionic surface charge and were mucoadhesive. In contrast, the surface charge of mucus-penetrating PLGA-PEG5k/CHA nanoparticles was near neutral, suggesting the formation of a dense PEG coating that masked the negative charge of PLGA and CHA. The concentration of low MW emulsifiers had no significant effect on nanoparticle surface charge and mucus penetrating property, likely because the PEG corona completely shields these molecules on the particle surface.

In addition, the inherent charge associated with the emulsifier (DSS and CHA are negatively charged, CTAB is positively charged, saponin, Vitamin-E TPGS, TWEEN20, TWEEN80 and SE are neutrally charged) had little or no effect on the surface charge and mucus penetrating property, further supporting the role of the PEG corona in shielding these low MW emulsifier molecules on the particle surface.

The choice of emulsifying agent also influenced the extent of PEG brush formation during the emulsification process. For example, while both PLGA-PEG5k/PVA nanoparticles and PLGA-PEG5k/CHA nanoparticles have near neutral surface charges, only the CHA formulations are mucus-penetrating. PVA and PEMA differ from the other emulsifiers as they contain a linear hydrophobic backbone decorated with hydrophilic side groups. It is possible that when PVA or PEMA stabilizes the oil/water interface, the hydrophobic polymer backbone can polyvalently adhere at the oil/water interface where they are in intimate contact with the protruding PEG brush. Thus, PVA and PEMA may disrupt the architecture of the PEG molecules on the particle surface, thereby rendering the particles mucoadhesive. In the case of PLGA-PEG5k/PEMA nanoparticles, the negative surface charge (-42 mV) suggests disruption of the PEG coating, as the charge likely originates from PEMA molecules exposed on the surface.

However, not all low MW emulsifiers are optimal for preparing MPP. For example, Cremophore EL, TWEEN 80, Vitamin-E TPGS, PLURONIC F127 and F68 were unable to fully stabilize the emulsion droplets during particle preparation, resulting in the formation of varying degrees of large aggregates. While the non-aggregated nanoparticle fractions thus prepared were mucus-penetrating, the nanoparticle yield was as low as 30%. Thus, the ability to produce MPPs from PEG-containing block copolymers by the emulsification method is critically dependent on both the MW and emulsification capability of the emulsifier. The emulsification capability of emulsifiers was estimated by the percentage of non-aggregated PLGA-PEG nanoparticles prepared in the aqueous phase containing 1% emulsifier. The emulsifiers must be strong enough to stabilize the emulsion droplets, yet be small enough to be completely shielded at the particle surface by the PEG corona.

PLGA-PEG/CHA nanoparticles with a wide range of PEG MWs (1, 2, 5, and 10 kDa), prepared by the emulsification method, all rapidly penetrated mucus. The nanoparticle surface charge was inversely proportional to the PEG MW and varied from -18 mV (1 kDa) to -2.3 mV (10 kDa). The surface PEG density [$\Gamma$] (number of PEG per 100 $nm^2$) measured by $^1$H NMR decreased as PEG MW increased. However, the ratio [$\Gamma/\Gamma^*$] of surface PEG density to the theoretical PEG density required for the formation of a brush-like PEG coating [$\Gamma^*$] was greater than 2, regardless of PEG MW, indicating the presence of a dense brush-like coating of PEG on the surface of PLGA-PEG(1-10 kDa)/CHA nanoparticles. The formation of a dense PEG brush on the particle surface appears to be necessary for mucus penetration.

It was confirmed that both hydrophobic and hydrophilic drugs can be efficiently encapsulated into PLGA-PEG MPP. Two model compounds: curcumin, a hydrophobic drug (MW=368 Da), and BSA, a hydrophilic protein (MW=66 kDa), were encapsulated using an o/w single emulsion into PLGA-PEG5k/CHA nanoparticles and a w/o/w double emulsion into PLGA-PEG5k/saponin nanoparticles, respectively. The encapsulation efficiency of both hydrophobic curcumin and hydrophilic BSA into MPP by using low MW emulsifiers was similar to that achieved with conventional particles (CP) by the emulsification method using PVA.

Curcumin and BSA-loaded nanoparticles rapidly diffused in mucus at rates only 6 and 36-fold slower than in water at $\tau$=1 s, respectively. On the other hand, nanoparticles prepared with PVA were immobilized in CVM, with transport rates more than 2,000-fold slower than in water. A substantial fraction, up to 40% and 30% of curcumin-MPP and BSA-MPP, respectively, are expected to penetrate physiologically thick mucus layers within 60 min, whereas < 1% of PVA-coated nanoparticles are expected to do so.

**III. Pharmaceutical compositions**

The formulations described herein contain an effective amount of nanoparticles in a pharmaceutical carrier appropriate for administration to a mucosal surface. The formulations can be administered parenterally (e.g., by injection or infusion), topically (e.g., to the eye), or via pulmonary administration.

**A. Pulmonary formulations**

Pharmaceutical formulations and their use for the pulmonary administration of active agents to patients are known in the art.

The respiratory tract is the structure involved in the exchange of gases between the atmosphere and the blood stream. The respiratory tract encompasses the upper airways, including the oropharynx and larynx, followed by the lower airways, which include the trachea followed by bifurcations into the bronchi and bronchioli. The upper and lower airways are called the conducting airways. The terminal bronchioli then divide into respiratory bronchioli which then lead to the ultimate respiratory zone, the alveoli, or deep lung, where the exchange of gases occurs.

Formulations can be divided into dry powder formulations and liquid formulations. Both dry powder and liquid formulations can be used to form aerosol formulations. The term aerosol as used herein refers to any preparation of a fine mist of particles, which can be in solution or a suspension, whether or not it is produced using a propellant.

**1. Dry Powder Formulations**

Dry powder formulations are finely divided solid formulations containing nanoparticle carriers which are suitable for pulmonary administration. Dry powder formulations include, at a minimum, one or more nanoparticle carriers which are suitable for pulmonary administration. Such dry powder formulations can be administered via pulmonary inhalation to a patient without the benefit of any carrier, other than air or a suitable propellant.

In other embodiments, the dry powder formulations contain one or more nanoparticle gene carriers in combination with a pharmaceutically acceptable carrier. In these embodiments, the nanoparticle gene carriers and pharmaceutical carrier can be formed into nano- or microparticles for delivery to the lung.

The pharmaceutical carrier may include a bulking agent or a lipid or surfactant. Natural surfactants such as dipalmitoylphosphatidylcholine (DPPC) are the most preferred. Synthetic and animal derived pulmonary surfactants include:

### Synthetic Pulmonary Surfactants

> Exosurf - a mixture of DPPC with hexadecanol and tyloxapol added as spreading agents
> Pumactant (Artificial Lung Expanding Compound or ALEC) - a mixture of DPPC and PG
> KL-4 - composed of DPPC, palmitoyl-oleoyl phosphatidylglycerol, and palmitic acid, combined with a 21 amino acid synthetic peptide that mimics the structural characteristics of SP-B.
> Venticute - DPPC, PG, palmitic acid and recombinant SP-C

### Animal derived surfactants

> Alveofact - extracted from cow lung lavage fluid
> Curosurf - extracted from material derived from minced pig lung
> Infasurf - extracted from calf lung lavage fluid
> Survanta - extracted from minced cow lung with additional DPPC, palmitic acid and tripalmitin
> Exosurf, Curosurf, Infasurf, and Survanta are the surfactants currently FDA approved for use in the U.S.

The pharmaceutical carrier may also include one or more stabilizing agents or dispersing agents. The pharmaceutical carrier may also include one or more pH adjusters or buffers. Suitable buffers include organic salts prepared from organic acids and bases, such as sodium citrate or sodium ascorbate. The pharmaceutical carrier may also include one or more salts, such as sodium chloride or potassium chloride.

Dry powder formulations are typically prepared by blending one or more nanoparticle carriers with one or more pharmaceutically acceptable carriers. Optionally, additional active agents may be incorporated into the mixture as discussed below. The mixture is then formed into particles suitable for pulmonary administration using techniques known in the art, such as lyophilization, spray drying, agglomeration, spray coating, coacervation, low temperature casting, milling (*e.g.,* air-attrition milling (jet milling), ball milling), high pressure homogenization, and/or supercritical fluid crystallization.

An appropriate method of particle formation can be selected based on the desired particle size, particle size distribution, and particle morphology desired for the formulation. In some cases, the method of particle formation is selected so as to produce a population of particles with the desired particle size, particle size distribution for pulmonary administration. Alternatively, the method of particle formation can produce a population of particles from which a population of particles with the desired particle size, particle size distribution for pulmonary administration is isolated, for example by sieving.

It is known in the art that particle morphology affects the depth of penetration of a particle into the lung. Accordingly, dry powder formulations is processed into particles having the appropriate mass median aerodynamic diameter (MMAD), tap density, and surface roughness to achieve delivery of the one or more active agents to the desired region(s) of the lung. For example, preferred particle morphologies for delivery to the deep lung are known in the art, and are described, for example, in U.S. Patent No. 7,052,678 to Vanbever, et al.

Particles having a mass median aerodynamic diameter (MMAD) of greater than about 5 microns generally do not reach the lung; instead, they tend to impact the back of the throat and are swallowed. Particles having diameters of about 3 to about 5 microns are small enough to reach the upper-to mid-pulmonary region (conducting airways), but may be too large to reach the alveoli. Smaller particles, (*i.e.,* about 0.5 to about 3 microns), are capable of efficiently reaching the alveolar region. Particles having diameters smaller than about 0.5 microns can also be deposited in the alveolar region by sedimentation.

The precise particle size range effective to achieve delivery to the alveolar region will depend on several factors, including the tap density of particles being delivered. Generally speaking, as tap density decreases, the MMAD of particles capable of efficiently reaching the alveolar region of the lungs increases. Therefore, in cases of particles with low tap densities, particles having diameters of about 3 to about 5 microns, about 5 to about 7 microns, or about 7 to about 9.5 microns can be efficiently delivered to the lungs. The preferred aerodynamic diameter for maximum deposition within the lungs can be calculated. See, for example, U.S. Patent No. 7,052,678 to Vanbever, et al.

Microparticles cannot diffuse through mucus even if their surface is muco-resistant. However, mucus-penetrating particles can be encapsulated in microparticles to impact upper lung, and subsequently release the nanoparticles. In some embodiments, the dry powder formulation is composed of a plurality of particles having a median mass aerodynamic diameter between about 0.05 to about 10 microns, more preferably betweeb about 0.05 microns to about 7 microns, most preferably between about 0.05 to about 5 microns. In some embodiments, the dry powder formulation is composed of a plurality of particles having a median mass aerodynamic diameter between about 0.05 microns to about 3 microns, more preferably between about 0.05 microns to about 1 micron, more preferably between about 0.05 microns to about 0.7 microns. In some embodiments, the dry powder formulation is composed of a plurality of particles having a median mass aerodynamic diameter between about 3 to about 5 microns. In some embodiments, the dry powder formulation is composed of a plurality of particles having a median mass aerodynamic diameter between about 5 to about 7 microns. In some embodiments, the dry powder formulation is composed of a plurality of particles having a median mass aerodynamic diameter between about 7 to about 9.5 microns.

In some cases, there may be an advantage to delivering particles larger than about 3 microns in diameter. Phagocytosis of particles by alveolar macrophages diminishes precipitously as particle diameter increases beyond about 3 microns. Kawaguchi, H., et al., Biomaterials 7: 61-66 (1986); Krenis, L. J. and Strauss, B., Proc. Soc. Exp. Med., 107: 748-750 (1961); and Rudt, S. and Muller, R. H., J. Contr. Rel, 22: 263-272 (1992). By administering particles with an aerodynamic volume greater than 3 microns, phagocytic engulfment by alveolar macrophages and clearance from the lungs can be minimized.

In some embodiments, at least about 80%, more preferably at least about 90%, most preferably at least about 95% of the particles in dry powder formulation have aerodynamic diameter of less than 10, 9, 8, 7, 6, or 5 microns. In some embodiments, at least about 80%, more preferably at least about 90%, most preferably at least about 95% of the particles in dry powder formulation have aerodynamic diameter of greater than about 0.03 microns.

In some embodiments, at least about 80%, more preferably at least about 90%, most preferably at least about 95% of the particles in dry powder formulation have aerodynamic diameter of greater than about 0.03 microns and less than about 10 microns, more preferably greater than about 0.03 microns and less than about 7 microns, most preferably greater than about 0.03 microns and less than about 5 microns. In some embodiments, at least about 80%, more preferably at least about 90%, most preferably at least about 95% of the particles in dry powder formulation have aerodynamic diameter of greater than about 0.03 microns and less than about 3 microns. In some embodiments, at least about 80%, more preferably at least about 90%, most preferably at least about 95% of the particles in dry powder formulation have aerodynamic diameter of greater than about 0.03 microns and less than about 5 microns. In some embodiments, at least about 80%, more preferably at least about 90%, most preferably at least about 95% of the particles in dry powder formulation have aerodynamic diameter of greater than about 0.03 microns and less than about 7 microns. In some embodiments, at least about 80%, more preferably at least about 90%, most preferably at least about 95% of the particles in dry powder formulation have aerodynamic diameter of greater than about 0.03 microns and less than about 9.5 microns.

In some embodiments, the particles have a tap density of less than about 0.4 $g/cm^3$, more preferably less than about 0.25 $g/cm^3$, most preferably less than about 0.1 $g/cm^3$. Features which can contribute to low tap density include irregular surface texture and porous structure.

In some cases, the particles are spherical or ovoid in shape. The particles can have a smooth or rough surface texture. The particles may also be coated with a polymer or other suitable material to control release of one or more active agents in the lungs.

Dry powder formulations can be administered as dry powder using suitable methods known in the art. Alternatively, the dry powder formulations can be suspended in the liquid formulation s described below, and administered to the lung using methods known in the art for the delivery of liquid formulations.


**2. Liquid Formulations**

Liquid formulations contain one or more nanoparticle carriers suspended in a liquid pharmaceutical carrier.

Suitable liquid carriers include, but are not limited to distilled water, de-ionized water, pure or ultrapure water, saline, and other physiologically acceptable aqueous solutions containing salts and/or buffers, such as phosphate buffered saline (PBS), Ringer's solution, and isotonic sodium chloride, or any other aqueous solution acceptable for administration to an animal or human.

Preferably, liquid formulations are isotonic relative to physiological fluids and of approximately the same pH, ranging e.g., from about pH 4.0 to about pH 7.4, more preferably from about pH 6.0 to pH 7.0. The liquid pharmaceutical carrier can include one or more physiologically compatible buffers, such as a phosphate buffers. One skilled in the art can readily determine a suitable saline content and pH for an aqueous solution for pulmonary administration.

Liquid formulations may include one or more suspending agents, such as cellulose derivatives, sodium alginate, polyvinylpyrrolidone, gum tragacanth, or lecithin. Liquid formulations may also include one or more preservatives, such as ethyl or *n*-propyl *p*-hydroxybenzoate.

In some cases the liquid formulation may contain one or more solvents that are low toxicity organic (i.e. non-aqueous) class 3 residual solvents, such as ethanol, acetone, ethyl acetate, tetrahydofuran, ethyl ether, and propanol.

These solvents can be selected based on their ability to readily aerosolize the formulation. Any such solvent included in the liquid formulation should not detrimentally react with the one or more active agents present in the liquid formulation. The solvent should be sufficiently volatile to enable formation of an aerosol of the solution or suspension. Additional solvents or aerosolizing agents, such as a freon, alcohol, glycol, polyglycol, or fatty acid, can also be included in the liquid formulation as desired to increase the volatility and/or alter the aerosolizing behavior of the solution or suspension.

Liquid formulations may also contain minor amounts of polymers, surfactants, or other excipients well known to those of the art. In this context, "minor amounts" means no excipients are present that might adversely affect uptake of the one or more active agents in the lungs.

### 3. Aerosol Formulations

The dry powder and liquid formulations described above can be used to form aerosol formulations for pulmonary administration. Aerosols for the delivery of therapeutic agents to the respiratory tract are known in the art. The term aerosol as used herein refers to any preparation of a fine mist of solid or liquid particles suspended in a gas. In some cases, the gas may be a propellant; however, this is not required. Aerosols may be produced using a number of standard techniques, including as ultrasonication or high pressure treatment.

Preferably, a dry powder or liquid formulation as described above is formulated into aerosol formulations using one or more propellants. Suitable propellants include air, hydrocarbons, such as pentane, isopentane, butane, isobutane, propane and ethane, carbon dioxide, chlorofluorocarbons, fluorocarbons, and combinations thereof. Suitable fluorocarbons include 1-6 hydrogen containing fluorocarbons, such as $CHF_2CHF_2$, $CF_3CH_2F$, $CH_2F_2CH_3$, and $CF_3CHFCF_3$ as well as fluorinated ethers such as $CF_3$-O-$CF_3$, $CF_2H$-O-$CHF_2$, and $CF_3$-$CF_2$-O-$CF_2$-$CH_3$. Suitable fluorocarbons also include perfluorocarbons, such as 1-4 carbon perfluorocarbons including $CF_3CF_3$, $CF_3CF_2CF_3$, and $CF_3CF_2CF_2CF_3$.

Preferably, the propellants include, but not limited to, one or more hydrofluoroalkanes (HFA). Suitable HFA propellants, include but are not limited to, 1,1,1,2,3,3,-heptafluoro-n-propane (HFA 227), 1,1,1,2-tetrafluoroethane (HFA 134) 1,1,1,2,25 3,3,3-heptafluoropropane (Propellant 227), or any mixture of these propellants.

Preferably, the one or more propellants have sufficient vapor pressure to render them effective as propellants. Preferably, the one or more propellants are selected so that the density of the mixture is matched to the density of the particles in the aerosol formulation in order to minimize settling or creaming of the particles in the aerosol formulation. The propellant is preferably present in an amount sufficient to propel a plurality of the selected doses of the aerosol formulation from an aerosol canister.

### 4. Devices for Pulmonary Administration

In some cases, a device is used to administer the formulations to the lungs. Suitable devices include, but are not limited to, dry powder inhalers, pressurized metered dose inhalers, nebulizers, and electrohydrodynamic aerosol devices.

Inhalation can occur through the nose and/or the mouth of the patient. Administration can occur by self-administration of the formulation while inhaling or by administration of the formulation via a respirator to a patient on a respirator.

*Dry Powder Inhalers*

The dry powder formulations described above can be administered to the lungs of a patient using a dry powder inhaler (DPI). DPI devices typically use a mechanism such as a burst of gas to create a cloud of dry powder inside a container, which can then be inhaled by the patient.

In a dry powder inhaler, the dose to be administered is stored in the form of a non-pressurized dry powder and, on actuation of the inhaler, the particles of the powder are inhaled by the subject. In some cases, a compressed gas (*i.e.*, propellant) may be used to dispense the powder, similar to pressurized metered dose inhalers (pMDIs). In some cases, the DPI may be breath actuated, meaning that an aerosol is created in precise response to inspiration. Typically, dry powder inhalers administer a dose of less than a few tens of milligrams per inhalation to avoid provocation of cough.

DPIs function via a variety of mechanical means to administer formulations to the lungs. In some DPIs, a doctor blade or shutter slides across the dry powder formulation contained in a reservoir, culling the formulation into a flowpath whereby the patient can inhale the powder in a single breath. In other DPIs, the dry powder formulation is packaged in a preformed dosage form, such as a blister, tabule, tablet, or gelcap, which is pierced, crushed, or otherwise unsealed to release the dry powder formulation into a flowpath for subsequent inhalation. Still others DPIs release the dry powder formulation into a chamber or capsule and use mechanical or electrical agitators to keep the dry powder formulation suspended in the air until the patient inhales.

Dry powder formulations may be packaged in various forms, such as a loose powder, cake, or pressed shape for insertion in to the reservoir of a DPI.

Examples suitable DPIs for the administration of the formulations described above include the Turbohaler® inhaler (Astrazeneca, Wilmington, Del.), the Clickhaler® inhaler (Innovata, Ruddington, Nottingham, UK), the Diskus® inhaler (Glaxo, Greenford, Middlesex, UK), the EasyHaler® (Orion, Expoo, FI), the Exubera® inhaler (Pfizer, New York, N.Y.), the Qdose® inhaler (Microdose, Monmouth Junction, N.J.), and the Spiros® inhaler (Dura, San Diego, Calif.).

*Pressurized Metered Dose Inhalers*

The liquid formulations described above can be administered to the lungs of a patient using a pressurized metered dose inhaler (pMDI).

Pressurized Metered Dose Inhalers (pMDIs) generally include at least two components: a canister in which the liquid formulation is held under pressure in combination with one or more propellants, and a receptacle used to hold and actuate the canister. The canister may contain a single or multiple doses of the formulation. The canister may include a valve, typically a metering valve, from which the contents of the canister may be discharged. Aerosolized drug is dispensed from the pMDI by applying a force on the canister to push it into the receptacle, thereby opening the valve and causing the drug particles to be conveyed from the valve through the receptacle outlet. Upon discharge from the canister, the liquid formulation is atomized, forming an aerosol.

pMDIs typically employ one or more propellants to pressurize the contents of the canister and to propel the liquid formulation out of the receptacle outlet, forming an aerosol. Any suitable propellants, including those discussed above, may be utilized. The propellant may take a variety of forms. For example, the propellant may be a compressed gas or a liquefied gas. Chlorofluorocarbons (CFC) were once commonly used as liquid propellants, but have now been banned. They have been replaced by the now widely accepted hydrofluororalkane (HFA) propellants.

pMDIs are available from a number of suppliers, including 3M Corporation, Aventis, Boehringer Ingleheim, Forest Laboratories, Glaxo-Wellcome, Schering Plough and Vectura. In some cases, the patient administers an aerosolized formulation by manually discharging the aerosolized formulation from the pMDI in coordination with inspiration. In this way, the aerosolized formulation is entrained within the inspiratory air flow and conveyed to the lungs.

In other cases, a breath-actuated trigger, such as that included in the Tempo® inhaler (MAP Pharmaceuticals, Mountain View, Calif.) may be employed that simultaneously discharges a dose of the formulation upon sensing inhalation. These devices, which discharge the aerosol formulation when the user begins to inhale, are known as breath-actuated pressurized metered dose inhalers (baMDIs).

*Nebulizers*

The liquid formulations described above can also be administered using a nebulizer. Nebulizers are liquid aerosol generators that convert the liquid formulation described able, usually aqueous-based compositions, into mists or clouds of small droplets, preferably having diameters less than 5 microns mass median aerodynamic diameter, which can be inhaled into the lower respiratory tract. This process is called atomization. The droplets carry the one or more active agents into the nose, upper airways or deep lungs when the aerosol cloud is inhaled. Any type of nebulizer may be used to administer the formulation to a patient, including, but not limited to pneumatic (jet) nebulizers and electromechanical nebulizers.

Pneumatic (jet) nebulizers use a pressurized gas supply as a driving force for atomization of the liquid formulation. Compressed gas is delivered through a nozzle or jet to create a low pressure field which entrains a surrounding liquid formulation and shears it into a thin film or filaments. The film or filaments are unstable and break up into small droplets that are carried by the compressed gas flow into the inspiratory breath. Baffles inserted into the droplet plume screen out the larger droplets and return them to the bulk liquid reservoir. Examples of pneumatic nebulizers include, but are not limited to, PARI LC Plus®, PARI LC Sprint®, Devilbiss PulmoAide®, and Boehringer Ingelheim Respima®.

Electromechanical nebulizers use electrically generated mechanical force to atomize liquid formulations. The electromechanical driving force can be applied, for example, by vibrating the liquid formulation at ultrasonic frequencies, or by forcing the bulk liquid through small holes in a thin film. The forces generate thin liquid films or filament streams which break up into small droplets to form a slow moving aerosol stream which can be entrained in an inspiratory flow.

In some cases, the electromechanical nebulizer is an ultrasonic nebulizer, in which the liquid formulation is coupled to a vibrator oscillating at frequencies in the ultrasonic range. The coupling is achieved by placing the liquid in direct contact with the vibrator such as a plate or ring in a holding cup, or by placing large droplets on a solid vibrating projector (a horn). The vibrations generate circular standing films which break up into droplets at their edges to atomize the liquid formulation. Examples of ultrasonic nebulizers include DuroMist®, Drive Medical Beetle Neb®, Octive Tech Densylogic®, and John Bunn Nano-Sonic®.

In some cases, the electromechanical nebulizer is a mesh nebulizer, in which the liquid formulation is driven through a mesh or membrane with small holes ranging from 2 to 8 microns in diameter, to generate thin filaments which break up into small droplets. In certain designs, the liquid formulation is forced through the mesh by applying pressure with a solenoid piston driver (for example, the AERx® nebulizer), or by sandwiching the liquid between a piezoelectrically vibrated plate and the mesh, which results in a oscillatory pumping action (for example EFlow®, AerovectRx®, or TouchSpray® nebulizer). In other cases, the mesh vibrates back and forth through a standing column of the liquid to pump it through the holes. Examples of such nebilzers include the AeroNeb Go®, AeroNeb Pro®. PARI EFlow®, Omron 22UE®; and Aradigm AERx®.

*Electrohydrodynamic Aerosol Devices*

The liquid formulations described above can also be administered using an electrohydrodynamic (EHD) aerosol device. EHD aerosol devices use electrical energy to aerosolize liquid drug solutions or suspensions. Examples of EHD aerosol devices are known in the art. See, for example, U.S. Patent No. 4,765,539 to Noakes et al. and U.S. Patent No. 4,962,885 to Coffee, R.A.

The electrochemical properties of the formulation may be important parameters to optimize when delivering the liquid formulation to the lung with an EHD aerosol device and such optimization is routinely performed by one of skill in the art.

## C. Parenteral Formulations

In some embodiments, the nanoparticles are formulated for parenteral delivery, such as injection or infusion, in the form of a solution or suspension. The formulation can be administered via any route, such as, the blood stream or directly to the organ or tissue to be treated. In some embodiments, the nanoparticles are formulated for parenteral formulation to the eye.

"Parenteral administration", as used herein, means administration by any method other than through the digestive tract or non-invasive topical or regional routes. For example, parenteral administration may include administration to a patient intravenously, intradermally, intraperitoneally, intrapleurally, intratracheally, intramuscularly, subcutaneously, subconjunctivally, by injection, and by infusion.

Parenteral formulations can be prepared as aqueous compositions using techniques is known in the art. Typically, such compositions can be prepared as injectable formulations, for example, solutions or suspensions; solid forms suitable for using to prepare solutions or suspensions upon the addition of a reconstitution medium prior to injection; emulsions, such as water-in-oil (w/o) emulsions, oil-in-water (o/w) emulsions, and microemulsions thereof, liposomes, or emulsomes.

The carrier can be a solvent or dispersion medium containing, for example, water, ethanol, one or more polyols (e.g., glycerol, propylene glycol, and liquid polyethylene glycol), oils, such as vegetable oils (e.g., peanut oil, corn oil, sesame oil, etc.), and combinations thereof. The proper fluidity can be maintained, for example, by the use of a coating, such as lecithin, by the maintenance of the required particle size in the case of dispersion and/or by the use of surfactants. In many cases, it will be preferable to include isotonic agents, for example, sugars or sodium chloride.

Solutions and dispersions of the active compounds as the free acid or base or pharmacologically acceptable salts thereof can be prepared in water or another solvent or dispersing medium suitably mixed with one or more pharmaceutically acceptable excipients including, but not limited to, surfactants, dispersants, emulsifiers, pH modifying agents, and combination thereof.

Suitable surfactants may be anionic, cationic, amphoteric or nonionic surface active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, Poloxamer® 401, stearoyl monoisopropanolamide, and polyoxyethylene hydrogenated tallow amide. Examples of amphoteric surfactants include sodium N-dodecyl-β-alanine, sodium N-lauryl-β-iminodipropionate, myristoamphoacetate, lauryl betaine and lauryl sulfobetaine.

The formulation can contain a preservative to prevent the growth of microorganisms. Suitable preservatives include, but are not limited to, parabens, chlorobutanol, phenol, sorbic acid, and thimerosal. The formulation may also contain an antioxidant to prevent degradation of the active agent(s).

The formulation is typically buffered to a pH of 3-8 for parenteral administration upon reconstitution. Suitable buffers include, but are not limited to, phosphate buffers, acetate buffers, and citrate buffers.

Water soluble polymers are often used in formulations for parenteral administration. Suitable water-soluble polymers include, but are not limited to, polyvinylpyrrolidone, dextran, carboxymethylcellulose, and polyethylene glycol.

Sterile injectable solutions can be prepared by incorporating the active compounds in the required amount in the appropriate solvent or dispersion medium with one or more of the excipients listed above, as required, followed by filtered sterilization. Generally, dispersions are prepared by incorporating the various sterilized active ingredients into a sterile vehicle which contains the basic dispersion medium and the required other ingredients from those listed above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The powders can be prepared in such a manner that the particles are porous in nature, which can increase dissolution of the particles. Methods for making porous particles are well known in the art.

Pharmaceutical formulations for ocular administration are preferably in the form of a sterile aqueous solution or suspension of particles formed from one or more polymer-drug conjugates. Acceptable solvents include, for example, water, Ringer's solution, phosphate buffered saline (PBS), and isotonic sodium chloride solution. The formulation may also be a sterile solution, suspension, or emulsion in a nontoxic, parenterally acceptable diluent or solvent such as 1,3-butanediol.

In some instances, the formulation is distributed or packaged in a liquid form. Alternatively, formulations for ocular administration can be packed as a solid, obtained, for example by lyophilization of a suitable liquid formulation. The solid can be reconstituted with an appropriate carrier or diluent prior to administration.

Solutions, suspensions, or emulsions for ocular administration may be buffered with an effective amount of buffer necessary to maintain a pH suitable for ocular administration. Suitable buffers are well known by those skilled in the art and some examples of useful buffers are acetate, borate, carbonate, citrate, and phosphate buffers.

Solutions, suspensions, or emulsions for ocular administration may also contain one or more tonicity agents to adjust the isotonic range of the formulation. Suitable tonicity agents are well known in the art and some examples include glycerin, mannitol, sorbitol, sodium chloride, and other electrolytes.

Solutions, suspensions, or emulsions for ocular administration may also contain one or more preservatives to prevent bacterial contamination of the ophthalmic preparations. Suitable preservatives are known in the art, and include polyhexamethylenebiguanidine (PHMB), benzalkonium chloride (BAK), stabilized oxychloro complexes (otherwise known as Purite®), phenylmercuric acetate, chlorobutanol, sorbic acid, chlorhexidine, benzyl alcohol, parabens, thimerosal, and mixtures thereof.

Solutions, suspensions, or emulsions for ocular administration may also contain one or more excipients known art, such as dispersing agents, wetting agents, and suspending agents.


## D. Topical Formulations

In still other embodiments, the nanoparticles are formulated for topical administration to mucosa. Suitable dosage forms for topical administration include creams, ointments, salves, sprays, gels, lotions, emulsions, liquids, and transdermal patches. The formulation may be formulated for transmucosal, transepithelial, transendothelial, or transdermal administration. The compositions contain one or more chemical penetration enhancers, membrane permeability agents, membrane transport agents, emollients, surfactants, stabilizers, and combination thereof.

In some embodiments, the, nanoparticles can be administered as a liquid formulation, such as a solution or suspension, a semi-solid formulation, such as an lotion or ointment, or a solid formulation. In some embodiments, the nanoparticles are formulated as liquids, including solutions and suspensions, such as eye drops or as a semi-solid formulation, such as ointment or lotion for topical application to mucosa, such as the eye or vaginally or rectally.

The formulation may contain one or more excipients, such as emollients, surfactants, emulsifiers, penetration enhancers, and the like.

"Emollients" are an externally applied agent that softens or soothes skin and are generally known in the art and listed in compendia, such as the "Handbook of Pharmaceutical Excipients", 4th Ed., Pharmaceutical Press, 2003. These include, without limitation, almond oil, castor oil, ceratonia extract, cetostearoyl alcohol, cetyl alcohol, cetyl esters wax, cholesterol, cottonseed oil, cyclomethicone, ethylene glycol palmitostearate, glycerin, glycerin monostearate, glyceryl monooleate, isopropyl myristate, isopropyl palmitate, lanolin, lecithin, light mineral oil, medium-chain triglycerides, mineral oil and lanolin alcohols, petrolatum, petrolatum and lanolin alcohols, soybean oil, starch, stearyl alcohol, sunflower oil, xylitol and combinations thereof. In one embodiment, the emollients are ethylhexylstearate and ethylhexyl palmitate.

"Surfactants" are surface-active agents that lower surface tension and thereby increase the emulsifying, foaming, dispersing, spreading and wetting properties of a product. Suitable non-ionic surfactants include emulsifying wax, glyceryl monooleate, polyoxyethylene alkyl ethers, polyoxyethylene castor oil derivatives, polysorbate, sorbitan esters, benzyl alcohol, benzyl benzoate, cyclodextrins, glycerin monostearate, poloxamer, povidone and combinations thereof. In one embodiment, the non-ionic surfactant is stearyl alcohol.

"Emulsifiers" are surface active substances which promote the suspension of one liquid in another and promote the formation of a stable mixture, or emulsion, of oil and water. Common emulsifiers are: metallic soaps, certain animal and vegetable oils, and various polar compounds. Suitable emulsifiers include acacia, anionic emulsifying wax, calcium stearate, carbomers, cetostearyl alcohol, cetyl alcohol, cholesterol, diethanolamine, ethylene glycol palmitostearate, glycerin monostearate, glyceryl monooleate, hydroxpropyl cellulose, hypromellose, lanolin, hydrous, lanolin alcohols, lecithin, medium-chain triglycerides, methylcellulose, mineral oil and lanolin alcohols, monobasic sodium phosphate, monoethanolamine, nonionic emulsifying wax, oleic acid, poloxamer, poloxamers, polyoxyethylene alkyl ethers, polyoxyethylene castor oil derivatives, polyoxyethylene sorbitan fatty acid esters,

polyoxyethylene stearates, propylene glycol alginate, self-emulsifying glyceryl monostearate, sodium citrate dehydrate, sodium lauryl sulfate, sorbitan esters, stearic acid, sunflower oil, tragacanth, triethanolamine, xanthan gum and combinations thereof. In one embodiment, the emulsifier is glycerol stearate.

Suitable classes of penetration enhancers are known in the art and include, but are not limited to, fatty alcohols, fatty acid esters, fatty acids, fatty alcohol ethers, amino acids, phospholipids, lecithins, cholate salts, enzymes, amines and amides, complexing agents (liposomes, cyclodextrins, modified celluloses, and diimides), macrocyclics, such as macrocylic lactones, ketones, and anhydrides and cyclic ureas, surfactants, N-methyl pyrrolidones and derivatives thereof, DMSO and related compounds, ionic compounds, azone and related compounds, and solvents, such as alcohols, ketones, amides, polyols (e.g., glycols). Examples of these classes are known in the art.

### i. Lotions, creams, gels, ointments, emulsions, and foams

"Hydrophilic" as used herein refers to substances that have strongly polar groups that readily interact with water.

"Lipophilic" refers to compounds having an affinity for lipids.

"Amphiphilic" refers to a molecule combining hydrophilic and lipophilic (hydrophobic) properties

"Hydrophobic" as used herein refers to substances that lack an affinity for water; tending to repel and not absorb water as well as not dissolve in or mix with water.

A "gel" is a colloid in which the dispersed phase has combined with the continuous phase to produce a semisolid material, such as jelly.

An "oil" is a composition containing at least 95% wt of a lipophilic substance. Examples of lipophilic substances include but are not limited to naturally occurring and synthetic oils, fats, fatty acids, lecithins, triglycerides and combinations thereof.

A "continuous phase" refers to the liquid in which solids are suspended or droplets of another liquid are dispersed, and is sometimes called the external phase. This also refers to the fluid phase of a colloid within which solid or fluid particles are distributed. If the continuous phase is water (or another hydrophilic solvent), water-soluble or hydrophilic drugs will dissolve in the continuous phase (as opposed to being dispersed). In a multiphase formulation (e.g., an emulsion), the discreet phase is suspended or dispersed in the continuous phase.

An "emulsion" is a composition containing a mixture of non-miscible components homogenously blended together. In particular embodiments, the non-miscible components include a lipophilic component and an aqueous component. An emulsion is a preparation of one liquid distributed in small globules throughout the body of a second liquid. The dispersed liquid is the discontinuous phase, and the dispersion medium is the continuous phase. When oil is the dispersed liquid and an aqueous solution is the continuous phase, it is known as an oil-in-water emulsion, whereas when water or aqueous solution is the dispersed phase and oil or oleaginous substance is the continuous phase, it is known as a water-in-oil emulsion. Either or both of the oil phase and the aqueous phase may contain one or more surfactants, emulsifiers, emulsion stabilizers, buffers, and other excipients. Preferred excipients include surfactants, especially non-ionic surfactants; emulsifying agents, especially emulsifying waxes; and liquid non-volatile non-aqueous materials, particularly glycols such as propylene glycol. The oil phase may contain other oily pharmaceutically approved excipients. For example, materials such as hydroxylated castor oil or sesame oil may be used in the oil phase as surfactants or emulsifiers.

An emulsion is a preparation of one liquid distributed in small globules throughout the body of a second liquid. The dispersed liquid is the discontinuous phase, and the dispersion medium is the continuous phase. When oil is the dispersed liquid and an aqueous solution is the continuous phase, it is known as an oil-in-water emulsion, whereas when water or aqueous solution is the dispersed phase and oil or oleaginous substance is the continuous phase, it is known as a water-in-oil emulsion. The oil phase may consist at least in part of a propellant, such as an HFA propellant. Either or both of the oil phase and the aqueous phase may contain one or more surfactants, emulsifiers, emulsion stabilizers, buffers, and other excipients. Preferred excipients include surfactants, especially non-ionic surfactants; emulsifying agents, especially emulsifying waxes; and liquid non-volatile non-aqueous materials, particularly glycols such as propylene glycol. The oil phase may contain other oily pharmaceutically approved excipients. For example, materials such as hydroxylated castor oil or sesame oil may be used in the oil phase as surfactants or emulsifiers.

A sub-set of emulsions are the self-emulsifying systems. These drug delivery systems are typically capsules (hard shell or soft shell) comprised of the drug dispersed or dissolved in a mixture of surfactant(s) and lipophilic liquids such as oils or other water immiscible liquids. When the capsule is exposed to an aqueous environment and the outer gelatin shell dissolves, contact between the aqueous medium and the capsule contents instantly generates very small emulsion droplets. These typically are in the size range of micelles or nanoparticles. No mixing force is required to generate the emulsion as is typically the case in emulsion formulation processes.

A "lotion" is a low- to medium-viscosity liquid formulation. A lotion can contain finely powdered substances that are in soluble in the dispersion medium through the use of suspending agents and dispersing agents. Alternatively, lotions can have as the dispersed phase liquid substances that are immiscible with the vehicle and are usually dispersed by means of emulsifying agents or other suitable stabilizers. In one embodiment, the lotion is in the form of an emulsion having a viscosity of between 100 and 1000 centistokes. The fluidity of lotions permits rapid and uniform application over a wide surface area.. Lotions are typically intended to dry on the skin leaving a thin coat of their medicinal components on the skin's surface.

A "cream" is a viscous liquid or semi-solid emulsion of either the "oil-in-water" or "water-in-oil type". Creams may contain emulsifying agents and/or other stabilizing agents. In one embodiment, the formulation is in the form of a cream having a viscosity of greater than 1000 centistokes, typically in the range of 20,000-50,000 centistokes. Creams are often time preferred over ointments as they are generally easier to spread and easier to remove.

The difference between a cream and a lotion is the viscosity, which is dependent on the amount/use of various oils and the percentage of water used to prepare the formulations. Creams are typically thicker than lotions, may have various uses and often one uses more varied oils/butters, depending upon the desired effect upon the skin. In a cream formulation, the water-base percentage is about 60-75 % and the oil-base is about 20-30 % of the total, with the other percentages being the emulsifier agent, preservatives and additives for a total of 100 %.

An "ointment" is a semisolid preparation containing an ointment base and optionally one or more active agents. Examples of suitable ointment bases include hydrocarbon bases (e.g., petrolatum, white petrolatum, yellow ointment, and mineral oil); absorption bases (hydrophilic petrolatum, anhydrous lanolin, lanolin, and cold cream); water-removable bases (e.g., hydrophilic ointment), and water-soluble bases (e.g., polyethylene glycol ointments). Pastes typically differ from ointments in that they contain a larger percentage of solids. Pastes are typically more absorptive and less greasy that ointments prepared with the same components.

A "gel" is a semisolid system containing dispersions of small or large molecules in a liquid vehicle that is rendered semisolid by the action of a thickening agent or polymeric material dissolved or suspended in the liquid vehicle. The liquid may include a lipophilic component, an aqueous component or both. Some emulsions may be gels or otherwise include a gel component. Some gels, however, are not emulsions because they do not contain a homogenized blend of immiscible components. Suitable gelling agents include, but are not limited to, modified celluloses, such as hydroxypropyl cellulose and hydroxyethyl cellulose; Carbopol homopolymers and copolymers; and combinations thereof. Suitable solvents in the liquid vehicle include, but are not limited to, diglycol monoethyl ether; alklene glycols, such as propylene glycol; dimethyl isosorbide; alcohols, such as isopropyl alcohol and ethanol. The solvents are typically selected for their ability to dissolve the drug. Other additives, which improve the skin feel and/or emolliency of the formulation, may also be incorporated. Examples of such additives include, but are not limited, isopropyl myristate, ethyl acetate, $C_{12}$-$C_{15}$ alkyl benzoates, mineral oil, squalane, cyclomethicone, capric/caprylic triglycerides, and combinations thereof.

Foams consist of an emulsion in combination with a gaseous propellant. The gaseous propellant consists primarily of hydrofluoroalkanes (HFAs). Suitable propellants include HFAs such as 1,1,1,2-tetrafluoroethane (HFA 134a) and 1,1,1,2,3,3,3-heptafluoropropane (HFA 227), but mixtures and admixtures of these and other HFAs that are currently approved or may become approved for medical use are suitable. The propellants preferably are not hydrocarbon propellant gases which can produce flammable or explosive vapors during spraying. Furthermore, the compositions preferably contain no volatile alcohols, which can produce flammable or explosive vapors during use.

Buffers are used to control pH of a composition. Preferably, the buffers buffer the composition from a pH of about 4 to a pH of about 7.5, more preferably from a pH of about 4 to a pH of about 7, and most preferably from a pH of about 5 to a pH of about 7. In a preferred embodiment, the buffer is triethanolamine.

Preservatives can be used to prevent the growth of fungi and microorganisms. Suitable antifungal and antimicrobial agents include, but are not limited to, benzoic acid, butylparaben, ethyl paraben, methyl paraben, propylparaben, sodium benzoate, sodium propionate, benzalkonium chloride, benzethonium chloride, benzyl alcohol, cetylpyridinium chloride, chlorobutanol, phenol, phenylethyl alcohol, and thimerosal.

In certain embodiments, it may be desirable to provide continuous delivery of one or more noscapine analogs to a patient in need thereof. For topical applications, repeated application can be done or a patch can be used to provide continuous administration of the noscapine analogs over an extended period of time.

**E. Enteral Formulations**

Suitable oral dosage forms include tablets, capsules, solutions, suspensions, syrups, and lozenges. Tablets can be made using compression or molding techniques well known in the art. Gelatin or non-gelatin capsules can prepared as hard or soft capsule shells, which can encapsulate liquid, solid, and semi-solid fill materials, using techniques well known in the art.

Formulations may be prepared using one or more pharmaceutically acceptable excipients, including diluents, preservatives, binders, lubricants, disintegrators, swelling agents, fillers, stabilizers, and combinations thereof.

Excipients, including plasticizers, pigments, colorants, stabilizing agents, and glidants, may also be used to form coated compositions for enteral administration. Delayed release dosage formulations may be prepared as described in standard references such as "Pharmaceutical dosage form tablets", eds. Liberman et. al. (New York, Marcel Dekker, Inc., 1989), "Remington - The science and practice of pharmacy", 20th ed., Lippincott Williams & Wilkins, Baltimore, MD, 2000, and "Pharmaceutical dosage forms and drug delivery systems", 6th Edition, Ansel et al., (Media, PA: Williams and Wilkins, 1995). These references provide information on excipients, materials, equipment and process for preparing tablets and capsules and delayed release dosage forms of tablets, capsules, and granules.

The nanoparticles may be coated, for example to delay release once the particles have passed through the acidic environment of the stomach. Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name EUDRAGIT® (Roth Pharma, Westerstadt, Germany), zein, shellac, and polysaccharides.

Diluents, also referred to as "fillers," are typically necessary to increase the bulk of a solid dosage form so that a practical size is provided for compression of tablets or formation of beads and granules. Suitable diluents include, but are not limited to, dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powdered sugar.

Binders are used to impart cohesive qualities to a solid dosage formulation, and thus ensure that a tablet or bead or granule remains intact after the formation of the dosage forms. Suitable binder materials include, but are not limited to, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone.

Lubricants are used to facilitate tablet manufacture. Examples of suitable lubricants include, but are not limited to, magnesium stearate, calcium stearate, stearic acid, glycerol behenate, polyethylene glycol, talc, and mineral oil.

Disintegrants are used to facilitate dosage form disintegration or "breakup" after administration, and generally include, but are not limited to, starch, sodium starch glycolate, sodium carboxymethyl starch, sodium carboxymethylcellulose, hydroxypropyl cellulose, pregelatinized starch, clays, cellulose, alginine, gums or cross linked polymers, such as crosslinked PVP (Polyplasdone® XL from GAF Chemical Corp).

Stabilizers are used to inhibit or retard drug decomposition reactions that include, by way of example, oxidative reactions. Suitable stabilizers include, but are not limited to, antioxidants, butylated hydroxytoluene (BHT); ascorbic acid, its salts and esters; Vitamin E, tocopherol and its salts; sulfites such as sodium metabisulphite; cysteine and its derivatives; citric acid; propyl gallate, and butylated hydroxyanisole (BHA).

### IV. Methods of making MPPs

Techniques for making nanoparticles are defined in the claims. Pharmaceutically acceptable excipients, including pH modifying agents, disintegrants, preservatives, and antioxidants, can optionally be incorporated into the particles during particle formation. As described above, one or more additional active agents can also be incorporated into the nanoparticle during particle formation.

Nanoparticles can be prepared by solvent evaporation and solvent removal methods.

### 1. Solvent Evaporation

In this method, the polymeric components of the nanoparticle gene carrier are dissolved in a volatile organic solvent, such as methylene chloride. The organic solution containing the polymer-drug conjugate is then suspended in an aqueous solution that contains a surface active agent such as poly(vinyl alcohol). The resulting emulsion is stirred until most of the organic solvent evaporated, leaving solid nanoparticles. The resulting nanoparticles are washed with water and dried overnight in a lyophilizer. Nanoparticles with different sizes and morphologies can be obtained by this method.

**2. Solvent Removal**

In this method, the components of the nanoparticle gene carrier are dispersed or dissolved in a suitable solvent. This mixture is then suspended by stirring in an organic oil (such as silicon oil) to form an emulsion. Solid particles form from the emulsion, which can subsequently be isolated from the supernatant.

The nanoparticles are prepared using an emulsification in method. In general, the particles are prepared by either o/w single emulsion or w/o/w double emulsion method as described in R. C. Mundargi et al, J. Control. Release 125, 193 (2008), M. Li et al., Int. J. Pharm. 363, 26 (2008), C. E. Astete and C. M. Sabliov, J. Biomater. Sci. Polymer Ed. 17, 247 (2006), and R. A. Jain, Biomaterials, 21, 2475 (2000). In this procedure, the polymer is dissolved in an organic solvent, such as dichloromethane, to form an oil phase. The oil phase is added to an aqueous solution of the emulsifier, typically under probe sonication for a period of time (e.g., 2 minutes) to form an emulsion. The emulsion is added to another large volume of the emulsifier with magnetic stirring to evaporate the organic solvent.

Nanoparticles are collected by centrifugation (e.g., 20,000 g for 25mins) after filtering through a 1 $\mu$m size membrane filter and thoroughly washed with water. To prepare the nanoparticles for fluorescence microscopy, a certain amount of AF555-labeled polymers were blended before the emulsification process. In the control experiment of nanoprecipitation method, PLGA45k-PEG5k solution in acetonitrile at concentration of 25mg/ml was slowly injected into DI water under magnetic stirring (700rpm). After the complete removal of organic solvent, nanoparticles were collected by the same procedure as described above.

The diameter (nm), polydispersity index (PDI) and surface charge ($\zeta$ potential, mV) of nanoparticles obtained from three repeat measurements by dynamic light scattering on Zetasizer Nano ZS90 (Malvern Instruments, Southborough, MA). Nanoparticles were dispersed in 10 mM NaCl solution (pH 7). The morphology of the nanoparticles was characterized by transmission electron microscopy (TEM) on H7600 TEM (Hitachi, Japan).

**V. Methods of using MPPs**

The data described herein highlights the numerous potential advantages of mucus penetrating nanoparticles prepared by the emulsification method for mucosal drug delivery applications. First, the dominantly used emulsifier PVA can be replaced with low MW emulsifiers to prepare biodegradable nanoparticles with similar drug encapsulation by the emulsification method. The nanoparticles exhibit high drug loadings, such as greater than 5% for hydrophobic drugs, such as curcumin, and greater than 10% for biomolecules. The surface-altering material (e.g., PEG) can enhance delivery of the nanoparticle to the site of interest since it creates a neutral or near neutral sirface charge which can enhance transport through fluids and materials *in vivo.*

For example, the nanoparticles described herein rapidly penetrate human mucus barriers, whereas PVA-coated nanoparticles are immobilized. Thus, the most widely used industrial production method for controlled release particles can be applied to manufacture MPPs for drug delivery applications.

Second, it is expected that MPPs prepared by the emulsification method can rapidly penetrate at other mucosal surfaces, such as eyes, nose, lungs, gastrointestinal tract, and more. CVM shares similarity in chemical content and rheological properties with other mucus fluids. Indeed, it has been observed that MPP prepared by the emulsification method can rapidly penetrate normal airway mucus collected during surgery and sputum expectorated by cystic fibrosis (CF) patients.

Third, challenging hydrophilic drugs, including proteins, peptides and nucleic acids, can be encapsulated into the nanoparticles by the emulsification method. For example, vaccine antigens (such as ovalbumin and tetanus toxoid) could be formulated into biodegradable nanoparticles for vaccination, such as MPPs for mucosal vaccination.

Fourth, hydrophobic drugs, which are difficult to dissolve in water-miscible organic solvents, could be successfully formulated into biodegradable nanoparticles, such as MPPs, by the emulsification method. Improved pharmacokinetics and therapeutic efficacy of hydrophobic drugs could be expected through the delivery of nanoparticles, such as mucosal drug delivery of MPPs.

The present invention will be further understood by reference to the following non-limiting examples.

**Examples**

Materials and Methods

Cholic acid sodium salt, TWEEN® 20, TWEEN® 80, hexadecyltrimethylammonium bromide (CTAB), dioctyl sulfosuccinate sodium (DSS), Polyoxyl 35 hydrogenated castor oil (Cremophor EL) and D-$\alpha$-tocopherol polyethylene glycol 1000 (Vitamin E-TPGS), were purchased from Sigma (St. Louis, MO).

Poly(vinyl alcohol) (Mw=25kDa with 88% hydrolysis and 6kDa with 80% hydrolysis),and poly(ethylene-maleic anhydride, 1:1 molar ratio) with Mw~400kDa were bought from PolySciences (Warrington, PA).

Sugar ester D1216 (SE) was a gift from Mitsubishi-Kagaku Foods Co. (Tokyo, Japan).

Alexa Fluor 555 cadaverine was purchased from Invitrogen (Grand Island, NY).

Poly(lactic-co-glycolic acid) (PLGA; LA:GA 50:50) with inherent viscosity of 0.15-0.25 dL/g (MW approximately 15kDa) was purchased from Lakeshore Biomaterials (Birmingham, AL). PLGA(LA:GA 50:50)-PEG copolymers with PEG MW of 10, 5, 2 and 1 kDa, PLA-PEG5k and PCL-PEG5k were custom-synthesized by the Jinan Daigang Biomaterial Co., Ltd, (Jinan, China) and characterized by $^1$H NMR and gel permeation chromatography (GPC). A Shimadzu apparatus equipped with a refractive index detector and two Waters Styragel® HR4 and HR5 columns were used. The analysis was performed at 35 °C, using tetrahydrofuran (THF) as the eluent, at a flow rate of 0.5 ml/min. GPC was calibrated with polystyrene standards (Sigma, St. Louis, MO).

The chemical composition and molecular weight (MW) of PLGA-PEG block copolymer were characterized by $^1$H NMR. Polymers were dissolved in $CDCl_3$ and $^1$H NMR spectra were recorded using a Bruker 400 REM instrument at 400 MHz. The $^1$H NMR spectra for copolymers in $CDCl_3$ are shown in Figure 3. Peaks of CH (5.22 ppm) from LA unit, $CH_2$ (4.83 ppm) from GA unit, and $CH_2CH_2$ (3.65ppm) from ethylene oxide unit were integrated, where $I_{5.22}$, $I_{4.83}$, $I_{3.65}$ are the integral intensities of the peaks at 5.22, 4.83 and 3.65ppm, respectively. Ratio of LA:GA was estimated as $I_{5.22}$ : $(I_{4.83}/2)$.

The MW of PLGA-PEG was estimated as follows:

$$(I_{3.65}/4)/(I_{4.83}/2) = (MW_{PEG}/44)/(MW_{GA}/58)$$

$$(I_{3.65}/4)/(I_{5.22}/1) = (MW_{PEG}/44)/(MW_{LA}/72)$$

$MW_{PLGA-PEG} = MW_{PEG} + (MW_{GA} + MW_{LA})$, where $MW_{PEG}$ is 1, 2, 5 and 10kDa.

Similarly, the molecular weight of PLA-PEG and PCL-PEG were estimated as follows:

$$(I_{3.65}/4)/(I_{5.22}/1) = (MW_{PEG}/44)/(MW_{LA}/72)$$

$$MW_{PLA-PEG} = MW_{PEG} + MW_{LA};$$

$$(I_{3.65}/4)/((I_{4.06} + I_{2.31})/4) = (MW_{PEG}/44)/(MW_{CL}/114)$$

$$MW_{PCL-PEG} = MW_{PEG} + MW_{CL}),$$

where $MW_{PEG}$ is 5 kDa, $I_{4.06}$ abd $I_{2.31}$ are the integral intensities of the peaks from PCL at 4.06 and 2.31 ppm, respectively.

Characteristics of various PEG-containing block copolymers are shown in Table 1.

**Table 1:** Characteristics of PEG-containing block copolymers

| Block polymer | PEG [kDa] | LA:GA[a] | PEG content [b] [%] | Mn[c] [kDa] | Mn[d] [kDa] | Mw[d] [kDa] | PDI[d] |
|---|---|---|---|---|---|---|---|
| PLGA-PEG10k | 10 | 54:46 | 21.6[e] | 46.3 | 23.6 | 38.3 | 1.62 |
| PLGA-PEG5k | 5 | 51:49 | 6.0 | 83.0 | 39.2 | 57.8 | 1.48 |
| PLGA-PEG2k | 2 | 52:48 | 6.3 | 31.8 | 19.0 | 27.7 | 1.46 |
| PLGA-PEG1k | 1 | 61:39 | 5.7 | 17.7 | 19.3 | 27.6 | 1.43 |
| PLA-PEG5k | 5 | 100:0 | 5.3 | 94.9 | 64.7 | 87.4 | 1.35 |

(continued)

| Block polymer | PEG [kDa] | LA:GA[a] | PEG content [b] [%] | Mn[c] [kDa] | Mn[d] [kDa] | Mw[d] [kDa] | PDI[d] |
|---|---|---|---|---|---|---|---|
| PCL-PEG5k | 5 | | 6.4 | 77.9 | 54.6 | 73.6 | 1.35 |

[a] The molar ratio of LA:GA was measured by comparing the $^1$H NMR integral intensity at 5.22ppm (-CH- on lactide), 1.59ppm (-CH$_3$ on lactide) and 4.83ppm (-CH$_2$- on glycolide).

[b] PEG content in the block copolymers were determined by $^1$H NMR.

[c] PLGA-PEG molecular weight (Mn) was determined by $^1$H NMR through comparing the integral at 5.22ppm (-CH- in lactide), 1.59ppm (-CH$_3$ on lactide), 4.83ppm (-CH$_2$- in glycolide) and 3.65ppm (-CH$_2$CH$_2$- in PEG) and by taking into account of the known Mn of PEG. For PCL-PEG, integrals at 4.06ppm (-O-CH$_2$-) and 2.31 ppm (-CH$_2$-CO-) were analyzed.

[d] Mn, Mw and polydispersity (PDI) were measured by GPC.

[e] PLGA-PEG 10kDa nanoparticles were made from the blending of PLGA15kDa with PLGA-PEG10kDa (21.6% PEG content) with overall PEG content in the nanoparticles at 6 wt%.

The total PEG content within nanoparticles was determined by 1H NMR using Bruker 400 REM instrument at 400 mHz. The freeze-dried nanoparticles were accurately weighed and dissolved in CDC13 containing 1 wt% hexadeuterodimethyl sulfoxide (TMS) as internal standard. The PEG content was determined by comparing to a PEG 5kDa calibration curve achieved from 1H NMR spectra using TMS as internal standard.

The tracking of fluorescently labeled nanoparticles in fresh human cervicovaginal mucus (CVM) was performed as published 39-40. Briefly, 0.6 $\mu$l of nanoparticles at suitable dilution was mixed into 20 $\mu$l mucus and incubated for 1 hour prior to microscopy. Movies were captured at a temporal resolution of 66.7 ms using a silicon-intensified target camera (VE-1000, Dage-MTI) mounted on an inverted epifluorescence microscope equipped with 100$\times$ oil-immersion objective lens. Trajectories for n>150 particles per experiment were extracted using MetaMorph software (Universal Imaging). Tracking movies (20s) were analyzed using metamorph software (Universal Imaging, Glendale, WI). Time averaged mean square displacement (MSD) and effective diffusivity for each particle were calculated as a function of time scale. Three experiments were performed for each condition. A one tailed, unequal variance Student's t-test was used to evaluate significance (P <0.05).

The ITC experiments were performed at 25 °C, using a VP-ITC microcalorimeter (MicroCal Inc., USA). Experiments were performed by injecting 2 mg/ml solution of mucin in DI water into 2mL sample cell containing nanoparticles with different PEG surface density at a concentration of 1 mg/ml in water with a stirring speed of 481 rpm. A total 28 injections were performed with a spacing of s and a reference power of $\mu$cal/s. The first injection of 2 $\mu$l mucin solution was followed by 27 injections of 10 $\mu$l of mucin solution. Binding isotherms were plotted and analyzed using Origin software, where the ITC measurements were fit to a one-site binding model. Stochoimetry was applied to calculate the binding content of mucin on nanoparticle surface, presented as mg mucin per m$^2$.

**Example 1. Preparation of nanoparticles**

Materials and Methods

Biodegradable nanoparticles were prepared by either o/w single emulsion or w/o/w double emulsion method as described in R. C. Mundargi et al, J. Control. Release 125, 193 (2008), M. Li et al., Int. J. Pharm. 363, 26 (2008), C. E. Astete and C. M. Sabliov, J. Biomater. Sci. Polymer Ed. 17, 247 (2006), and R. A. Jain, Biomaterials, 21, 2475 (2000).

Nanoparticles were characterized for size, surface property and drug loading (for drug encapsulated nanoparticles). The displacements of nanoparticles were tracked in fresh, undiluted human CVM using multiple particle tracking.

*Nanoparticles prepared with different amounts of PEG*

PLGA-PEG nanoparticles were prepared with varying target PEG contents (0, 2, 3, 5, 8, 10 and 25 wt%, referred to as PLGA, PLGA-PEG2%, PLGA-PEG3%, PLGA-PEG5%, PLGA-PEG8%, PLGA-PEG10% and PLGA-PEG25%) using emulsification. The PEG molecular weight 5 kDa was selected since at the same PEG content, 6wt% PLGA-PEG nanoparticles with PEG ranging from 1 kDa to 10 kDa all can rapidly penetrate mucus. The target PEG contents were controlled by varying the ratio of PLGA and PLGA-PEG during the preparation of nanoparticles. Particle sizes of nanoparticles were controlled to around 100 nm by tuning polymer concentration and emulsification procedure, and all nanoparticles exhibited mono-dispersed diameter with small polydispersity index (less than 0.1) under dynamic light scattering. The nanoparticles were spherically shaped based on TEM study, and PLGA-PEG25% nanoparticles with the highest target PEG content showed less contrast at particle boundaries which probably resulted from the high content of lower electron-density PEG located at the surface.

Results

Table 2 shows the characteristics of the particles prepared as described above. The nanoparticles shown in Table 2, with 0% and 2% PEG are not in the scope of the claims.

**Table 2:** Nanoparticle Characteristics

| Target PEG content (wt%) | Diameter [nm] [a] | PDI [a] | $\zeta$-potential [mV] | $\alpha$ [b] | $D_w/D_m$ [c] |
|---|---|---|---|---|---|
| 25 | 91±5 | 0.094 | -2.7±0.7 | 0.89 | 6.0 |
| 10 | 117±7 | 0.097 | -2.4±0.6 | 0.80 | 8.7 |
| 8 | 116±8 | 0.068 | -4.3±0.9 | 0.81 | 7.7 |
| 5 | 106±6 | 0.085 | -7.0±0.7 | 0.78 | 17 |
| 3 | 101±6 | 0.078 | -10±0.1 | 0.53 | 142 |
| 2 | 91±6 | 0.075 | -20±1.4 | 0.31 | 4,000 |
| 0 | 144±6 | 0.056 | -72±2.2 | 0.13 | 38,000 |

[a] Diameter and polydispersity index (PDI) of nanoparticles are measured by the dynamic laser scattering.
[b] The transport rate also can be reflected by the slope $\alpha$ of double logarithmic MSD versus time scale plots ($\alpha$ =1 represents unobstructed Brownian transport whereas smaller $\alpha$ reflects increased obstruction to particle movement.)
[c] Ratios of the ensemble average diffusion coefficients in mucus ($D_m$) compared to in water ($D_w$) for nanoparticles, and effective diffusivity values were calculated at a time scale of 1 s.

Data are means±SD.

Increasing the target PEG content resulted in a substantial decrease of nanoparticle surface charge (Table 2), and nearly neutral surface charge (approximately 4mV) was achieved when PEG contents reached 8wt% and above. Decreased surface charge reflects the increased surface PEG coverage because dense PEG coatings can effectively shield the surface charge of nanoparticles. However, surface charge (zeta-potential) measurement is not able to provide quantitative information for assessing PEG surface density with regard to the number of PEG chains on the surface of a particle. Furthermore, surface charge measurement can be affected by the core materials and the measurement media. $^1$H NMR was utilized to directly quantify the PEG surface density on nanoparticles. As shown in Table 3, surface PEG content on nanoparticles increases with the increase in the target PEG content. Table 3 shows the PEG surface density of PLGA-PEG nanoparticles with different PEG contents. Surface PEG level was detected by $^1$H NMR in $D_2O$ as compared to a standard DSS (1 wt%). The total PEG content in nanoparticles was measured by $^1$H NMR in $CDCl_3$ as compared to a standard TMS (1 wt%). N/A, not applicable.

Table 3: Surface PEG Content on Nanoparticles

| Target PEG content (wt%) | Total PEG content in the whole NP (wt%) | PEG content on NP surface (wt%) | PEG surface density [$\Gamma$] (chains/100nm$^2$) [a] | [$\Gamma/\Gamma$*] [b] |
|---|---|---|---|---|
| 25 | 13.0±0.3 | 12.9±1.0 | 29.7±2.9 | 6.7±0.7 |
| 10 | 7.4±0.1 | 7.2±0.2 | 19.4±1.3 | 4.4±0.3 |
| 8 | 6.0±0.3 | 6.0±0.3 | 16.4±1.6 | 3.7±0.4 |
| 5 | 3.7±0.1 | 3.7±0.2 | 10.4±0.2 | 2.4±0.04 |
| 3 | 2.5±0.1 | 2.6±0.1 | 6.5±0.2 | 1.5±0.05 |
| 2 | 1.4±0.4 | 1.4±0.02 | 3.3±0.1 | 0.76±0.02 |
| 0 | N/A | N/A | N/A | N/A |

[a] PEG density [$\Gamma$] means the calculated number of PEG molecules per 100 nm$^2$ by assuming that all PEG chains on surface are full length of PEG 5kDa.
[b] PEG density/full surface coverage [$\Gamma/\Gamma$*]. Full mushroom coverage [$\Gamma$*] means the number of unconstrained PEG molecules per 100 nm$^2$. (value < 1 indicates mushroom coverage with low PEG density, whereas > 1 represents brush regime; when the value >>1 represents a dense brush regime with very high PEG density).
Data (mean±SD) are the average of at least three different batches of samples.

**Example 2: Nanoparticles prepared with different emulsifiers**

Materials and Methods

Alexa Fluor 555 cadaverine (AF555) was chemically conjugated to polymers. Nanoparticles were prepared using emulsification. Typically, a mixture (total 50 mg) of PLGA-PEG5k and AF555-labeled PLGA-PEG5k was dissolved in 1mL dichloromethane (DCM). The oil phase was poured into 5mL aqueous solution containing 1% emulsifier under sonication (VibraCell, Sonics & Materials Inc., Newtown, CT) at 30% amplitude for 2 mins in an ice-water bath to form the oil-in-water emulsion.

The emulsion was poured into another 40mL aqueous phase of emulsifier solution under magnetic stirring at 700 rpm for at least 3 hours to allow the solvent to evaporate. The solvent was further evaporated by placing the solution in a vacuum chamber for 30 mins. The final nanoparticle suspensions were filtered through 1 $\mu$m syringe filter, centrifuged at 20,000 g for 25 mins and thoroughly washed with water.

Emulsifiers including cholic acid sodium salt (CHA), dioctyl sulfosuccinate sodium (DSS), hexadecyltrimethyl ammonium bromide (CTAB), polyvinyl alcohol (PVA), poly(ethylene-maleic anhydride) (PEMA), Saponin, TWEEN20, TWEEN80 and sugar ester D1216 (SE) were tested at a concentration of 1% w/v. CHA solutions at 0.01%-0.5% w/v were also able to make nanoparticles successfully. PLURONIC® F127, F68 solutions and other low MW emulsifiers, like Cremophor EL and Vitamin-E TPGS, were also tested, but unstable emulsions resulted in large aggregated particles.

Table 4 shows the characteristics of nanoparticles prepared using PLGA-PEG (Mn~83kDa) and PLGA (Mn~15kDa) and various emulsifiers (1% w/v).

**Table 4.** Characteristics of biodegradable nanoparticles prepared by the emulsification method using PLGA-PEG5k (Mn ~83kDa) and PLGA (Mn ~15kDa) and representative emulsifiers (1% w/v). The emulsifiers having molecular weight equal or more to 1500 amu are not in the scope of the claims.

| Polymer | Emulsifier | Emulsifier MW [Da] | Diameter [nm] | $\zeta$-potential [mV] | $D_w/D_m$ [a] |
|---|---|---|---|---|---|
| PLGA-PEG5k | DSS | 444 | 136±5 | -5.5±0.5 | 3.9 |
| | CHA | 430 | 115±11 | -3.7±0.4 | 5.1 |
| | CTAB | 364 | 77±3 | -4.6±0.7 | 5.6 |
| | Saponin | 1.8k | 108±1 | -7.0±0.7 | 10 |
| | SE | 540 | 97±3 | -4.2±0.3 | 6.8 |
| | TWEEN®20 | 1.2k | 156±7 | -3.8±0.3 | 3.5 |
| | TWEEN®80 | 1.3k | 152±6 | -4.2±0.3 | 6.8 |
| | F127 | 12.5k | 169±8 | -2.4±0.2 | 4.2 |
| | F68 | 8.4k | 162±5 | -3.3±0.3 | 4.2 |
| | TPGS | 1.5k | 204±7 | -4.8±0.3 | 5.6 |
| | Cremophor | 2.1k | 232±4 | -3.5±0.1 | 3.6 |
| | PVA | 25k | 156±8 | -2.9±0.3 | 40,000 |
| | PEMA | 400k | 185±6 | -42±1.6 | 23,000 |
| PLGA | PVA | 25k | 175±5 | -2.6±1.0 | 19,000 |
| | CHA | 430 | 144±6 | -72±2.2 | 41,000 |

[a] Ratios of the ensemble average diffusion coefficients in water ($D_w$) compared to in mucus ($D_m$) at a time scale of 1 s.

To evaluate the effect of polyethylene glycol molecular weight (PEG MW) on mucus-penetrating property of nanoparticles prepared by the emulsification, CHA was selected as the representative low MW strong emulsifier. PLGA-PEG nanoparticles with different PEG MWs at approximately 6wt% PEG content were prepared in 0.5% CHA solution. In order to achieve overall 6wt% PEG content for PLGA-PEG10k nanoparticles, blends of PLGA-PEG10k (21.6 wt%) and PLGA15k were utilized.

Results

Properties of nanoparticles prepared from PEG of various molecular weights (~6wt% PEG content) are shown in Table 5.

**Table 5.** Characteristics of biodegradable nanoparticles prepared using PEG of various MWs (~6wt% PEG content) by the emulsifcation method. The examples in the two last lines of table 5 are not in the scope of the claims.

| PEG MW [kDa] | Diameter [nm] | $\zeta$-potential [mV] | PEG density [$\Gamma$] [a] (#PEG/100nm$^2$) | [$\Gamma/\Gamma$*] [b] | $D_w/D_m$ |
|---|---|---|---|---|---|
| 10 | 124 ± 6 | -2.3 ± 0.1 | 6.7 | 3.0 | 9.6 |
| 5 | 107 ± 3 | -4.2 ± 0.3 | 13.9 | 3.3 | 4.4 |
| 2 | 128 ± 1 | -12 ± 0.9 | 26.2 | 2.5 | 5.0 |
| 1 | 134 ± 5 | -18 ± 1.2 | 45.0 | 2.3 | 7.7 |

[a] PEG density [$\Gamma$] indicates the number of PEG molecules per 100 nm$^2$. The surface PEG content was quantified by $^1$H NMR of nanoparticles in $D_2O$.

[b] Ratio of PEG density to full surface coverage [$\Gamma/\Gamma$*]. Full surface coverage [$\Gamma$*] indicates the theoretical number of unconstrained PEG molecules required to fully coat a 100 nm$^2$ surface. ([$\Gamma/\Gamma$*]<1 indicates mushroom regime with low surface PEG density, whereas >1 represents brush regime with high surface PEG density)

Properties of nanoparticles prepared from various concentrations of CHA and PLGA-PEG5k containing 6wt% PEG are shown in Table 6.

**Table 6:** Characterization of biodegradable nanoparticles using different concentration of emulsifier (CHA) prepared by the emulsification method. PLGA-PEG5k containing 6wt% PEG was used.

| Emulsifier [w/v %] | Diameter [nm] | $\zeta$-potential [mV] | $D_w/D_m$ |
|---|---|---|---|
| 1 | 115±10 | -3.7±0.4 | 5.1 |
| 0.5 | 107±3 | -4.2±0.3 | 4.4 |
| 0.1 | 142±9 | -3.5±0.6 | 4.1 |
| 0.01 | 125±6 | -5.1±0.5 | 4.3 |

**Example 3: Preparation of Drug Encapsulated Nanoparticles**

Materials and Methods

Curcumin was selected as a model hydrophobic drug which was dissolved with polymer in DCM. The procedure was similar to that for preparation of unloaded nanoparticles. The prepared curcumin-nanoparticles can be visualized in mucus because of curcumin's intrinsic fluorescence.

BSA was used as a model hydrophilic drug because it is representative of large molecule biologics. BSA-FITC and BSA (10% ratio of BSA-FITC) were dissolved in 0.2mL 16% w/v aqueous solution at 37°C. This solution was added to 1mL of 100mg/ml PLGA-PEG5k in DCM solution during probe sonication (30% amplitude, 1min with Is pulse) in the ice-water bath. The resultant W/O primary emulsion was immediately added to a second water phase (5mL 1% saponin solution) under sonication (20% amplitude for 2 min). The double emulsion was transferred to another 40 mL 1% saponin solution with magnetic stirring for 3 hours. Nanoparticles were filtered through 1$\mu$m syringe filer, washed and collected by centrifugation. BSA-FITC allowed the possibility to track BSA-loaded nanoparticles in mucus.

Results

The target drug loading for curcumin nanoparticle and BSA nanoparticle was 9.1% and 16.7%, respectively.

**Example 4. Estimation of emulsification capability**

Materials and Methods

PLGA-PEG5k (MW approximately 83kDa) was used as the model polymer and was dissolved in DCM at 50mg/ml. A 0.5ml solution of PLGA-PEG5k in DCM was added to 5ml aqueous phase containing 1% (w/v) emulsifiers under sonication with 30% amplitude to prepare emulsion using the same method described above. The formed emulsion was added to an additional 20ml 1% emulsifier solution under magnetic stirring at 700 rpm for 3 hours. The emulsification capability of each emulsifier was estimated by its ability to prevent the formation of aggregated particles. Aggregated particles were collected by centrifugation at 500g for 20min, and the remaining nanoparticles in the supernatant were collected by centrifugation at 30,000g for 25min. The weight ratio of nanoparticles to aggregated particles was calculated, and used as the index to estimate the emulsification capability of the emulsifier.

*Diameter and surface charge*

Diameter and $\xi$-potential (surface charge) of nanoparticles were measured using Zetasizer Nano ZS90. Nanoparticles were resuspended in 10mM NaCl solution. TEM samples were prepared by dropping a dilute suspension of nanoparticle on a TEM grid and allowed to air dry. Particle morphology was characterized using a H7600 transmission electron microscope (Hitachi, Japan).

*Encapsulation efficiency*

The encapsulation efficiency of curcumin in nanoparticles was measured by dissolving the freeze-dried nanoparticles in DMSO and measuring the absorbance at 430nm using Biotek Synergy MX plate reader. The drug content was determined by comparing to the curcumin calibration curve (concentration range 0-50 $\mu$g/ml). Absorbance of blank nanoparticle in DMSO at the same polymer concentration was subtracted. The encapsulation efficiency of BSA-FITC was analyzed after alkaline digestion. A known amount of freeze-dried nanoparticles underwent complete hydrolysis in 1M sodium hydroxide. The resultant solution was analyzed using Biotek Synergy MX plate reader at 490nm excitation wavelength and 525nm emission wavelength. Standard solutions containing the same amount of polymer and increasing amounts of BSA-FITC at the same processing condition were prepared. The amount of BSA in the nanoparticles was determined by comparison to the BSA-FITC calibration curve.

Drug loading (DL) and encapsulation efficiency (EE) were calculated as follows:

$$DL\ (\%) = \frac{Weight\ of\ drug}{Weight\ of\ nanoparticles} \times 100\%$$

$$EE\ (\%) = \frac{Experimental\ drug\ loading}{Target\ drug\ loading} \times 100\%$$

Results

The results for various emulsifiers are shown in Table 7. The examples with PVA as emulsifier are not in the scope of the claims.

Table 7: The encapsulation of model hydrophobic drug (curcumin) and model hydrophilic drug (BSA) in both MPP (CHA and saponin as emulsifier) and CP (PVA as emulsifier) using PLGA-PEG5k (6wt% PEG)

| Formulation | DL [%] [a] | EE [%] [b] | Diameter [nm] | $\zeta$-potential [mV] | $D_w/D_m$ |
|---|---|---|---|---|---|
| PLGA-PEG5k/CHA (curcumin) | 4.5 | 49 | 156$\pm$12 | -5.1$\pm$0.5 | 6 |
| PLGA-PEG5k/PVA (curcumin) | 4.3 | 47 | 151$\pm$11 | -3.3$\pm$1.1 | 2400 |
| PLGA-PEG5k/Saponin (BSA) | 11.4 | 68 | 164$\pm$1 | -4.5+0.4 | 36 |
| PLGA-PEG5k/PVA (BSA) | 11.5 | 69 | 218$\pm$22 | -2.2$\pm$0.8 | 5100 |

[a] Drug loading (DL%) represents the weight content of drug in nanoparticles.
[b] Drug encapsulation efficiency (EE%) represents the ratio of final drug loading in comparison to the theoretical drug loading.

*Quantification of surface polyethylene glycol (PEG) density*

The surface PEG density on nanoparticles was determined by 1H NMR using Bruker 400 REM instrument at 400 MHz. Relaxation time was set at 10s, and ZG at 90°. Nanoparticles with different PEG content were directly prepared in 0.5% CHA $D_2O$ solution and suspended in $D_2O$ with 1wt% 3-(trimethylsilyl)-1-propanesulfonic acid, sodium salt as internal standard for $^1$H NMR analysis.

A known weight of PEG 5kDa (Sigma, St Louis, MO) homopolymer in $D_2O$ with 1% 3-(trimethylsilyl)-1-propanesulfonic acid, sodium salt was serially diluted to different concentration to set up the calibration curve for the PEG signal in $^1$H NMR, and this calibration curve was used to calculate the surface PEG content on nanoparticles.

A 0.2ml solution of nanoparticles in $D_2O$ was lyophilized and weighed. By assuming all surface PEG chains were full length of PEG 5kDa, the surface PEG density was calculated as the number of PEG molecules per 100 $nm^2$ surface area on nanoparticles. Control $^1$H NMR experiments were also performed with PLGA nanoparticles prepared by the same method and there were no detectable CHA peaks for PLGA nanoparticles. PEG density, [Γ], is the number of PEG molecules on nanoparticle surface per 100 $nm^2$. It can be calculated by dividing the total PEG content (MPEG, in mole) detected by $^1$H NMR by the total surface area of all nanoparticles as follows:

$$[\Gamma] = \frac{(M_{PEG} \times 6.02 \times 10^{23})}{W_{NP}/d_{NP}/4/3\pi(D/2)^3} \div 4\pi(D/2)^2 \times 100$$

where $W_{NP}$ is the total mass of nanoparticles, $d_{NP}$ is the density of nanoparticle (the density of nanoparticles is assumed to be equal to the density of polymer, 1.21g/ml for PLGA), and D is the particle diameter as measured by the dynamic light scattering.

Full surface mushroom coverage [Γ*] is the number of unconstrained PEG molecules occupying 100 $nm^2$ of particle surface area. In order to determine [Γ*], the surface area occupied by a single PEG chain was estimated. Using random-walk statistics, a single PEG chain occupies an area at the interface given by a sphere of diameter $\xi$.

$$\xi = 0.76m^{0.5} \, [\text{Å}]$$

where m is the molecular weight of the PEG chain. The surface area occupied by one PEG molecule can be determined from $(\xi/2)^2$. Thus PEG 5kDa has an unconstrained molecule sphere with diameter of 5.4nm, and occupies a surface area of 22.7 $nm^2$. Therefore, the number of PEG molecules to fully cover 100 $nm^2$ surface area, [Γ*], is 4.4.

[Γ/Γ*] can be used as an index to measure the PEG density on the nanoparticle surface, where the values <1 indicates low PEG density where PEG molecules are in a mushroom conformation; whereas values>1 indicate high PEG density where PEG molecules are in a brush-like conformation. Similarly, [Γ*] for PEG 10kDa, 2 kDa and 1 kDa is 2.2, 11 and 22, respectively. The results are shown in Table 2 above.

Table 8 show the PEG surface density of PLGA-PEG nanoparticles with different PEG contents. Surface PEG level was detected by 1H NMR in D2O as compared to a standard DSS (1 wt%). The total PEG content in nanoparticles was measured by 1H NMR in CDC13 as compared to a standard TMS (1 wt%). N/A, not applicable.

**Table 8.** PEG surface density of PLGA-PEG nanoparticles with different PEG contents

| Target PEG content (wt%) | Total PEG content in the whole NP (wt%) | PEG content on NP surface (wt%) | PEG surface density [Γ] (chains/100nm$^2$) [a] | [Γ/Γ*] [b] |
|---|---|---|---|---|
| 25 | 13.0+0.3 | 12.9±1.0 | 29.7±2.9 | 6.7±0.7 |
| 10 | 7.4±0.1 | 7.2±0.2 | 19.4±1.3 | 4.4±0.3 |
| 8 | 6.0±0.3 | 6.0±0.3 | 16.4±1.6 | 3.7±0.4 |
| 5 | 3.7±0.1 | 3.7±0.2 | 10.4±0.2 | 2.4±0.04 |
| 3 | 2.5±0.1 | 2.6±0.1 | 6.5±0.2 | 1.5±0.05 |
| 2 | 1.4±0.4 | 1.4±0.02 | 3.3±0.1 | 0.76±0.02 |
| 0 | N/A | N/A | N/A | N/A |

[a] PEG density [Γ] means the calculated number of PEG molecules per 100 $nm^2$ by assuming that all PEG chains on surface are full length of PEG 5kDa.

[b] PEG density/full surface coverage [Γ/Γ*]. Full mushroom coverage [Γ*] means the number of unconstrained PEG molecules per 100 $nm^2$. (< 1 indicates mushroom coverage with low PEG density, whereas > 1 represents brush regime; when the value >>1 represents a dense brush regime with very high PEG density).

Data (mean±SD) are the average of at least three different batches of samples.

The surface PEG density ($[\Gamma]$, the number of PEG chains per 100 nm$^2$) was calculated and compared with full surface mushroom coverage ($[\Gamma^*]$, the number of unconstrained PEG molecules per 100 nm$^2$). PLGA-PEG3% nanoparticles showed a surface PEG content of 2.6wt% with density of 6.5 PEG/100nm$^2$, equal to $[\Gamma]/[\Gamma^*]$=1.5, which rendered PLGA-PEG3% with brush conformation of surface PEG coating. High dense brush conformation of PEG coating ($[\Gamma]/[\Gamma^*]$>3) was achieved at PEG surface density higher than 10 PEG/100nm$^2$ (PLGA-PEG5%).

By dissolving the freeze-dried PLGA-PEG nanoparticles in NMR solvent CDCl$_3$, the total PEG content within nanoparticles by $^1$H NMR was measured and it was found that the total PEG contents in nanoparticles (both surface PEG and the PEG embedded within nanoparticle cores) was very close to the surface PEG contents, as shown in Table 5. Almost all the PEG chains in the PLGA-PEG nanoparticles prepared by the emulsification method were detected on the particle surface. The emulsification method involved the evaporation of organic solvent (dichloromethane) from the emulsion droplets and the followed solidification of polymer cores. The slow evaporation of organic solvent provides enough time for hydrophilic PEG chain to diffuse and assemble at the surface of nanoparticles, which resulted in the high partition ratio of PEG to the surface. However, there is significant loss of PEG during the preparation of nanoparticles by the emulsification method, and the PEG loss ratio can be as high as 50% for PLGA-PEG25% nanoparticles.

Similar to previous reports, the loss of PEG may be due to the formation of micelles by the low molecular weight portion of PLGA-PEG in the copolymer, which have higher PEG content and higher hydrophilicity. This part of very small sized particles containing higher PEG content polymers cannot be collected after centrifugation and washing steps, which can be confirmed from the increased average molecular weight of polymer after the formation of nanoparticles in comparison to the raw polymer, measured by gel permeation chromatography. PLGA-PEG10% nanoparticles (117 nm) prepared by nanoprecipitation method (solvent diffusion method) in a control experiment showed 6.5wt% total PEG content in the nanoparticle and only 89% of PEG chains were detected at surface (equal to 5.8wt% surface PEG content).

**Example 5. Mucus penetrating tracking of nanoparticles**

Materials and Methods

Human cervical vaginal mucus (CVM) was collected. Briefly, undiluted cervicovaginal secretions from women with normal vaginal flora were obtained using a self-sampling menstrual collection device following a protocol approved by the Institutional Review Board of the Johns Hopkins University. The device was inserted into the vagina for 60s, removed and placed into a 50ml centrifuge tube and centrifuged at 1000rpm for 2 min to collect the secretions.

The tracking of fluorescently labeled nanoparticles in fresh human cervicovaginal mucus (CVM) was performed. Briefly, 0.6 $\mu$l of nanoparticles at suitable dilution was added to 20 $\mu$l mucus within a custom-made chamber slide and incubated at room temperature for 1 hour prior to microscopy. The trajectories of nanoparticles in CVM were recorded by using multiple particle tracking (MPT). 20s movies were captured at a temporal resolution of 66.7ms using a silicon-intensified target camera (VE-1000, Dage-MTI) mounted on an inverted epifluorescence microscope equipped with 100x oil-immersion objective (N.A., 1.3). Tracking movies (20s) were analyzed using MetaMorph software (Universal Imaging, Glendale, WI).

Time averaged mean square displacement (MSD) and effective diffusivity for each particle were calculated as a function of time scale:

$$<\Delta r2(\tau)>=[x(t+\tau)-x(t)]^2+[y(t+\tau)-y(t)]^2$$

where x and y represent the nanoparticle coordinates as a funciton of time and $\tau$ is the time lag.

Curcumin-loaded nanoparticles and FITC-BSA-loaded nanoparticles were tracked in human CVM in the same manner using the fluorescence from either encapsulated curcumin or BSA-FITC. Particle penetration into a mucus layer was modeled using Fick's second law and diffusion coefficients obtained from tracking experiments.

Results

The comparison of transport of human CVM of PLA-PEG and PCL-PEG nanoparticles containing CHA and PVA prepared by emulsification is shown in Figures 1a-h. Figures 1a and 1b show representative trajectories of PLA-PEG and PCL-PEG nanoparticles containing CHA and PVA. Figures 1c and 1d are graphs showing ensemble-averaged geometric mean square displacements (<MSD>) as function of time scale. Figures 1e and 1f are graphs showing the distributions of the logarithms of individual particle effective diffusivities (Deff) at a time scale of 1 s. Figures 1g and 1h are graphs showing the estimated fraction of particles capable of penetrating a physiological 30$\mu$m thick mucus layer over time. Data represent three independent experiments with $\geq$ 120 nanoparticles tracked for each experiment. Error bars are presented as s.e.m. This data shows immobilization of nanoparticles made using PVA and rapid mucus penetration for nanoparticles made using a low MW emulsifier, CHA, with effective diffusivities similar to those measured for PLGA-PEG5k nanoparticles.

The effect of PEG molecular weight on transport rate of MPPs in CVM is shown in Figures 2a and 2b. Figures 2a and 2b show the effect of PEG MW on transport rate of MPP in human cervicovaginal mucus. Figure 2a is a graph showing the ensemble-averaged geometric mean square displacement <MSD> as a function of time scale. Figure 2b is a graph showing the distributions of the logarithms of individual particle effective diffusivities (Deff) at a time scale of 1 s. Particles were prepared with the emulsification method using PLGA-PEG (6wt% PEG). Data represent three independent experiments with ≥ 120 nanoparticles tracked for each experiment. Error bars are presented as s.e.m. These particles all rapidly penetrated mucus (see also Table 5).

The nanoparticle surface charge was inversely proportional to the PEG MW and varied from -18 mV (1 kDa) to -2.3 mV (10 kDa). The surface PEG density [Γ] (number of PEG per 100 nm2) measured by 1H NMR decreased as PEG MW increased. However, the ratio [Γ/Γ*][11] of surface PEG density to the theoretical PEG density required for the formation of a brush-like PEG coating [Γ*] was greater than 2 (Table 5), regardless of PEG MW, indicating the presence of a dense brush-like coating of PEG on the surface of PLGA-PEG(1-10 kDa)/CHA nanoparticles.

Figures 3a-3c shows the transport rates of curcumin and BSA loaded MPPs and conventional particles (CPs). Figure 3a is a graph showing the ensemble-averaged geometric mean square displacement <MSD> as a function of time scale. Figure 3b is a graph showing the distributions of the logarithms of individual particle effective diffusivities (Deff) at a time scale of 1 s. Figure 3c is a graph showing the estimated fraction of particles predicted to be capable of penetrating a 30 $\mu$m thick mucus layer over time. Data represent three independent experiments with ≥ 120 nanoparticles tracked for each experiment. Error bars are presented as s.e.m. Curcumin and BSA-loaded nanoparticles rapidly diffused in mucus at rates only 6 and 36-fold slower than in water at $\tau$=1 s, respectively (Figure 3a). In contrast, nanoparticles prepared with PVA were immobilized in CVM (Figure 3b), with transport rates more than 2,000-fold slower than in water.

PLGA nanoparticles without PEG coating were completely immobilized within mucus with diffusivities 38,000 times slower than the diffusivities of same sized nanoparticles in water. The presence of PEG surface coating on nanoparticles significantly improved their diffusion through the highly viscoelastic mucus, PLGA-PEG3% with surface PEG density of 6.5 PEG/100nm2 showed increased Dw/Dm value up to 142. Further increasing the surface PEG density up to 10.4 PEG/100nm2, PLGA-PEG5% nanoparticles were only 17-fold slower than their diffusion in water. More than 90% of the nanoparticles were diffusive when surface PEG density was higher than 16.4 PEG/100nm2 (PLGA-PEG8%). Further increase of the surface PEG density likely will not significantly improve the particle diffusivity within mucus, since a surface density of 16.4 PEG/100nm2 is already capable to efficiently shield the binding of mucus components. Approximately 50-70% nanoparticles of PLGA-PEG8%, 10% and 25% were able to penetrate physiological 30$\mu$m thick mucus layer within 60 mins, are much higher rates than PLGA-PEG5%, PLGA-PEG3% (dense coating), PLGA-PEG2%(low coating) and PLGA (no coating).

## Example 6: Stability of Nanoparticles in Mucus.

### Materials and Methods

The stability of nanoparticles in mucus by minimizing the adhesive interaction between particles and mucus components is an important criterion for their application as mucus-penetration drug carriers *in vivo*. The change in nanoparticle size in the presence of mucin as the indication of mucin binding was studied to determine the stability of nanoparticles with different PEG surface density at the presence of mucin. Mucin extracted from bovine submaxillary gland was chosen as a model mucin because mucin is the main component of mucus and mucin from bovine submaxillary gland shares similarity with human CVM in both structure and physiological properties.

Nanoparticles were incubated with mucin solution (10mg/ml) and the change of particle size over time was monitored.

### Results

PLGA-PEG nanoparticles with PEG surface density ≥16.4 PEG/100nm2 were stable in mucin solution retaining their hydrodynamic diameter during the whole 3 hour incubation, and at these PEG surface densities the PEG coatings were in high dense brush conformation ([Γ]/[Γ*]>3). In contrast, PLGA-PEG5% nanoparticles with surface density of 6.5 PEG/100nm2 showed approximately 5% increase in particle diameter after the incubation with mucin solution even only for 5 mins, and the PEG surface density on these PLGA-PEG5% nanoparticles already resulted in a brush PEG coating ([Γ]/[Γ*]>1). Therefore, brush PEG coatings alone are not enough to completely shield the mucin binding. There was a progressive increase in particle size with decreased PEG surface density from brush conformation to mushroom conformation. Without PEG coating, PLGA nanoparticles exhibited a dramatic size increase from 109±2 nm to 207±9 nm within 5 min of incubation in mucin.

Figure 4a is a schematic illustrating the influence of surface PEG coverage ([Γ/Γ*]) on mucus penetration of nanoparticles. The upper panels show the preparation of PLGA-PEG nanoparticles with surface PEG coating at increasing coverage. As surface PEG coverage increases, PEG regime changes from mushroom (neighboring PEG chains do not overlap, [Γ/Γ*] <1, Figure 4a), to brush (neighboring PEG chains overlap, 1<[Γ/Γ*]<3, Figure 4b), to dense brush ([Γ/Γ*]>3, Figure 4c). The middle panels illustrate how PEG coverage determines the muco-adhesive interaction after mucus exposure. At low PEG coverage ([Γ/Γ*] <1), mucin fibers strongly adhere to nanoparticle core. At middle PEG coverage (1<[Γ/Γ*]<3), mucin fibers still can partially absorb to the nanoparticle core. At high ([Γ/Γ*]>3) PEG coverage, the nanoparticle cores were completely shielded by the bioinert PEG corona resulting in no absorption of mucin to nanoparticles. The lower panels show that nanoparticles with low PEG coverage are immobilized in mucus, nanoparticles with middle PEG coverage are hindered or even immobilized in mucus, nanoparticles with high and very high PEG coverage are able to rapidly penetrate mucus.

## Claims

1. Nanoparticles formed by emulsion of one or more core polymers, one or more surface altering materials, and one or more low molecular weight emulsifiers having a molecular weight less than 1500 amu,

    wherein the one or more surface altering materials are selected from the group consisting of polyethylene glycol (PEG) and poloxamer, or wherein the particles are formed from block copolymers containing PEG,
    wherein the nanoparticle possess a ζ-potential of between 10 mV and -10 mV when dispersed in 10 mM NaCl solution at pH 7;
    wherein the nanoparticles further comprise one or more therapeutic, prophylactic, or diagnostic agents, and
    wherein the nanoparticles penetrate cervicovaginal mucus (CVM) with effective speeds less than 25-fold slower than the same particles in water.

2. The nanoparticles of claim 1 wherein the one or more surface altering materials are poloxamers.

3. The nanoparticles of claim 1, wherein the core polymer and the surface altering material are distinct components.

4. The nanoparticles of claim 1, wherein the surface altering material is covalently bound to the core polymer.

5. The nanoparticles of claim 3, wherein the core polymer is a block copolymer containing one or more blocks of the surface altering material.

6. The nanoparticles of claim 4, wherein the core polymer comprises a single block of the surface altering material covalently bound at one end of the core polymer.

7. The nanoparticles of any one of claims 1, 3, 4, or 5, wherein the core polymer further comprises one or more polymers that are not covalently bound to the surface altering material

8. The nanoparticles of claim 6, wherein the one or more polymers not covalently bound to the surface altering material have the same chemical composition as the one or more core polymers.

9. The nanoparticles of claim 1, wherein the surface altering material is polyethylene glycol.

10. The nanoparticles of claim 9, wherein the molecular weight of the polyethylene glycol is from 1kD to 10kD, preferably from 1kD to 5kD, more preferably 5kD.

11. The nanoparticles of claim 9 or 10 wherein the density of polyethylene glycol, when measured by $^1$H NMR is from 5 to 50 chains/100nm$^2$.

12. The nanoparticles of any one of claims 1-11, wherein the molecular weight of the one or more emulsifiers is less than 1300, 1200, 1000, 800, 600, or 500 amu.

13. The nanoparticles of claim 12, wherein the one or more emulsifiers are neutral, positively charged, negatively charged, or combinations thereof.

14. The nanoparticles of claim 13, wherein the one or more low molecular weight emulsifiers are selected from the group consisting of cholic acid sodium salt, dioctyl sulfosuccinate sodium, hexadecyltrimethyl ammonium bromide, saponin, TWEEN 20, TWEEN 80, and sugar esters.

15. The nanoparticles of any preceding claim, wherein the nanoparticles penetrate cervicovaginal mucus (CVM) with effective speeds less than 20, 19, 18, 17, 16, 15, 14, 13, 12, 11, 10, 9, 8, 7, 6, 5 or 4-fold slower than the same particles in water.

16. The nanoparticles of any one of claims 1-15, wherein the one or more emulsifiers have an emulsification capability of at least 50%, preferably at least 55, 60, 65, 70, 75, 80, 85, 90, or 95%.

17. A pharmaceutical composition comprising the nanoparticles of claim 16 and one or more pharmaceutically acceptable carriers.

18. A composition comprising an effective amount of the particles of claim 16 for use as a medicament.

19. The composition of claim 18, wherein the particles are administered enterally, parenterally, or topically.

20. The composition of claim 19, wherein the particles are administered by intravenous, subcutaneous, intramuscular, or intraperitoneal injection.

21. The composition of claim 19, wherein the particles are administered subconjunctivally.

22. The composition of claim 21, wherein the particles are topically applied to the eye or a compartment thereof.

23. The composition of claim 21, wherein the particles are administered to the pulmonary tract, intranasally, intravaginally, rectally, or buccally.

24. A method of making the particles of any one of claims 1-16, the method comprising dissolving the one or more core polymers in an organic solvent, adding the solution of the one or more core polymers to an aqueous solution or suspension of the emulsifier to form an emulsion, and adding the emulsion to a second solution or suspension of the emulsifier to effect formation of the nanoparticles.

25. Nanoparticles prepared from the method of claim 24.

26. Nanoparticles according to any of claims 1 to 16 for use in therapy to penetrate mucus barriers.

**Patentansprüche**

1. Nanopartikel, die durch Emulsion aus einem oder mehreren Kernpolymeren gebildet sind, und ein oder mehrere oberflächenverändernde Materialien und ein oder mehrere niedermolekulare Emulgatoren mit einem Molekulargewicht von weniger als 1500 amu, wobei das eine oder die mehreren oberflächenverändernden Materialien aus der Gruppe bestehend aus Polyethylenglykol (PEG) und Poloxamer ausgewählt sind oder wobei die Partikel aus PEG-enthaltenden Blockcopolymeren gebildet sind, wobei die Nanopartikel ein $\zeta$-Potential zwischen 10 mV und -10 mV besitzen, wenn sie in einer 10 mM NaCl-Lösung bei einem pH-Wert von 7 dispergiert sind; wobei die Nanopartikel ferner ein oder mehrere therapeutisches, prophylaktisches oder diagnostisches Mittel umfassen und wobei die Nanopartikel den zervikovaginalen Schleim (CVM) mit effektiven Geschwindigkeiten durchdringen, um weniger als das 25-fache langsamer als die gleichen Partikel in Wasser sind.

2. Nanopartikel nach Anspruch 1, wobei die eine oder mehreren oberflächenverändernden Materialien Poloxamere sind.

3. Nanopartikel nach Anspruch 1, wobei das Kernpolymer und das oberflächenverändernde Material unterschiedliche Komponenten sind.

4. Nanopartikel nach Anspruch 1, wobei das oberflächenverändernde Material kovalent an das Kernpolymer gebunden ist.

5. Nanopartikel nach Anspruch 3, wobei das Kernpolymer ein Blockcopolymer ist, das einen Block oder mehrere Blöcke des oberflächenverändernden Materials enthält.

6. Nanopartikel nach Anspruch 4, wobei das Kernpolymer einen einzelnen Block des oberflächenverändernden Materials umfasst, welcher an einem Ende des Kernpolymers kovalent gebunden ist.

7. Nanopartikel nach einem der Ansprüche 1,3, 4 oder 5, wobei das Kernpolymer ferner ein oder mehrere Polymere, die nicht kovalent an das oberflächenverändernde Material gebunden sind.

8. Nanopartikel nach Anspruch 6, wobei das eine oder die mehreren Polymere, die nicht kovalent an das oberflächenverändernde Material gebunden sind, die gleiche chemische Zusammensetzung wie das eine oder die mehreren Kernpolymere aufweisen.

9. Nanopartikel nach Anspruch 1, wobei das oberflächenverändernde Material Polyethylenglykol ist.

10. Nanopartikel nach Anspruch 9, wobei das Molekulargewicht des Polyethylenglykols 1 kD bis 10 kD, vorzugsweise 1 kD bis 5 kD, stärker bevorzugt 5 kD beträgt.

11. Nanopartikel nach Anspruch 9 oder 10, wobei, wenn durch $^1$H NMR gemessen, die Dichte des Polyethylenglykol 5 bis 50 Ketten/100 $nm^2$ beträgt.

12. Nanopartikel nach einem der Ansprüche 1 bis 11, wobei das Molekulargewicht des einen oder der mehreren Emulgator(en) weniger als 1300, 1200, 1000, 800, 600 oder 500 amu beträgt.

13. Nanopartikel nach Anspruch 12, wobei der eine oder die mehreren Emulgator(en) neutral, positiv geladen, negativ geladen oder Kombinationen davon ist/sind.

14. Nanopartikel nach Anspruch 13, wobei der eine oder die mehreren niedermolekularen Emulgatoren ausgewählt sind aus der Gruppe bestehend aus Cholsäurenatriumsalz, Dioctylsulfosuccinatnatrium, Hexadecyltrimethyl-Ammoniumbromid, Saponin, TWEEN 20, TWEEN 80 und Zuckerestern.

15. Nanopartikel nach einem der vorangehenden Ansprüche, wobei die Nanopartikel den zervikovaginalen Schleim (CVM) mit effektiven Geschwindigkeiten durchdringen, die um weniger als das 20, 19, 18, 17, 16, 15, 14, 13, 12, 11, 10, 9, 8, 7, 6, 5 oder 4-fache langsamer als die gleichen Partikel in Wasser sind.

16. Nanopartikel nach einem der Ansprüche 1 bis 15, wobei der eine oder die mehreren Emulgatoren ein Emulgiervermögen von mindestens 50 %, vorzugsweise mindestens 55, 60, 65, 70, 75, 80, 85, 90 oder 95 % aufweisen.

17. Pharmazeutische Zusammensetzung, umfassend Nanopartikel nach Anspruch 16 und einen oder mehrere pharmazeutisch verträgliche Träger.

18. Zusammensetzung umfassend, eine wirksame Menge des Partikels nach Anspruch 16 zur Verwendung als Medikament.

19. Zusammensetzung nach Anspruch 18, wobei die Partikel enteral, parenteral oder topisch verabreicht werden.

20. Zusammensetzung nach Anspruch 19, wobei die Partikel durch intravenöse, subkutane, intramuskuläre oder intraperitoneale Injektion verabreicht werden.

21. Zusammensetzung nach Anspruch 19, wobei die Partikel subkonjunktival verabreicht werden.

22. Zusammensetzung nach Anspruch 21, wobei die Partikel topisch auf das Auge oder ein Bereich davon aufgetragen werden.

23. Zusammensetzung nach Anspruch 21, wobei die Partikel in dem Lungentrakt intranasal, intravaginal, rektal oder bukkal verabreicht werden.

24. Verfahren zum Herstellen der Partikel nach einem der Ansprüche 1 bis 16, wobei das Verfahren Folgendes umfasst:

Auflösen des einen oder der mehreren Kernpolymere in einem organischen Lösungsmittel, Zugabe der Lösung des einen oder der mehreren Kernpolymere zu einer wässrigen Lösung oder Suspension des Emulgators zum Bilden einer Emulsion und Zugeben der Emulsion zu einer zweiten Lösung oder Suspension des Emulgators, um die Bildung der Nanopartikel zu bewirken.

**25.** Nanopartikel, die nach dem Verfahren von Anspruch 24 vorbereitet sind.

**26.** Nanopartikel nach einem der Ansprüche 1 bis 16 zur Verwendung in der Therapie zur Penetration von Schleimbarrieren.

**Revendications**

**1.** Nanoparticules formées par émulsion d'un ou de plusieurs polymères de cœur, d'une ou de plusieurs matières de modification de surface et d'un ou de plusieurs émulsifiants de faible masse moléculaire ayant une masse moléculaire inférieure à 1 500 amu, la ou les matières de modification de surface étant choisies dans le groupe constitué par le polyéthylène glycol (PEG) et de poloxamère, ou les particules étant formées à partir de copolymères séquencés contenant du PEG, la nanoparticule possédant un potentiel $\zeta$ compris entre 10 et -10 mV lorsqu'elle est dispersée dans une solution de NaCl à 10 mM à un pH de 7 ;
les nanoparticules comprenant en outre un ou plusieurs agents thérapeutiques, prophylactiques ou diagnostiques, et les nanoparticules pénétrant dans le mucus cervico-vaginal (MCV) à des vitesses effectives inférieures à 25 fois plus lentes que les mêmes particules pénétrant dans de l'eau.

**2.** Nanoparticules selon la revendication 1, dans lesquelles la ou les matières de modification de surface sont des poloxamères.

**3.** Nanoparticules selon la revendication 1, dans lesquelles le polymère de cœur et la matière de modification de surface sont des composants distincts.

**4.** Nanoparticules selon la revendication 1, dans lesquelles la matière de modification de surface est liée par covalence au polymère de cœur.

**5.** Nanoparticules selon la revendication 3, dans lesquelles le polymère de cœur est un copolymère séquencé contenant une ou plusieurs séquences de la matière de modification de surface.

**6.** Nanoparticules selon la revendication 4, dans lesquelles le polymère de cœur comprend une seule séquence de la matière de modification de surface liée par covalence à une extrémité du polymère de cœur.

**7.** Nanoparticules selon l'une quelconque des revendications 1, 3, 4 ou 5, dans lesquelles le polymère de cœur comprend en outre un ou plusieurs polymères qui ne sont pas liés par covalence à la matière de modification de surface.

**8.** Nanoparticules selon la revendication 6, dans lesquelles le ou les polymères non liés par covalence à la matière de modification de surface ont la même composition chimique que le ou les polymères de cœur.

**9.** Nanoparticules selon la revendication 1, dans lesquelles la matière de modification de surface est du polyéthylène glycol.

**10.** Nanoparticules selon la revendication 9, dans lesquelles la masse moléculaire du polyéthylène glycol est comprise entre 1 et 10 kD, de préférence entre 1 et 5 kD, plus préférablement de 5 kD.

**11.** Nanoparticules selon la revendication 9 ou 10, dans lesquelles la densité du polyéthylène glycol, lorsqu'elle est mesurée par RMN du $^1$H, est comprise entre 5 et 50 chaînes/100 nm$^2$.

**12.** Nanoparticules selon l'une quelconque des revendications 1 à 11, dans lesquelles la masse moléculaire du ou des émulsifiants est inférieure à 1 300, 1 200, 1 000, 800, 600 ou 500 amu.

**13.** Nanoparticules selon la revendication 12, dans lesquelles le ou les émulsifiants sont neutres, chargés positivement, chargés négativement, ou présentent des combinaisons de ces caractéristiques.

14. Nanoparticules selon la revendication 13, dans lesquelles le ou les émulsifiants de faible masse moléculaire sont choisis dans le groupe constitué par le sel de sodium de l'acide cholique, le dioctyl sulfosuccinate de sodium, le bromure d'hexadécyltriméthyl ammonium, la saponine, le TWEEN 20, le TWEEN 80 et les esters de sucre.

15. Nanoparticules selon l'une quelconque des revendications précédentes, les nanoparticules pénétrant dans le mucus cervico-vaginal (MCV) à des vitesses efficaces inférieures à 20, 19, 18, 17, 16, 15, 14, 13, 12, 11, 10, 9, 8, 7, 6, 5 ou 4 fois plus lentes que les mêmes particules pénétrant dans de l'eau.

16. Nanoparticules selon l'une quelconque des revendications 1 à 15, dans lesquelles le ou les émulsifiants ont une capacité d'émulsification d'au moins 50 %, de préférence d'au moins 55, 60, 65, 70, 75, 80, 85, 90 ou 95 %.

17. Composition pharmaceutique comprenant des nanoparticules selon la revendication 16 et un ou plusieurs supports pharmaceutiquement acceptables.

18. Composition comprenant une quantité efficace des particules selon la revendication 16, destinée à être utilisée comme médicament.

19. Composition selon la revendication 18, dans laquelle les particules sont administrées par voie entérale, parentérale ou topique.

20. Composition selon la revendication 19, dans laquelle les particules sont administrées par injection intraveineuse, sous-cutanée, intramusculaire ou intrapéritonéale.

21. Composition selon la revendication 19, dans laquelle les particules sont administrées par voie sous-conjonctivale.

22. Composition selon la revendication 21, dans laquelle les particules sont appliquées par voie topique sur l'œil ou sur un compartiment de celui-ci.

23. Composition selon la revendication 21, dans laquelle les particules sont administrées aux voies pulmonaires, par voie intranasale, intravaginale, rectale ou buccale.

24. Procédé de préparation des particules selon l'une quelconque des revendications 1 à 16, le procédé consistant à dissoudre le ou les polymères de cœur dans un solvant organique, à ajouter la solution du ou des polymères de cœur à une solution ou suspension aqueuse de l'émulsifiant de façon à former une émulsion, et à ajouter l'émulsion à une seconde solution ou suspension de l'émulsifiant pour réaliser la formation des nanoparticules.

25. Nanoparticules préparées au moyen du procédé selon la revendication 24.

26. Nanoparticules selon l'une quelconque des revendications 1 à 16, destinées à être utilisées en thérapie pour pénétrer les barrières du mucus.

PLA-PEG5k/CHA

1 μm

*FIG. 1A*

PLA-PEG5k/PVA

1 μm

100 nm

PCL-PEG5k/CHA

1 μm

*FIG. 1B*

PCL-PEG5k/PVA

1 μm

100 nm

—— PLA-PEG/CHA
---- PLA-PEG/PVA

$\langle MSD \rangle / \mu m^2$

Time Scale/s

*FIG. 1C*

—— PCL-PEG/CHA
---- PCL-PEG/PVA

$\langle MSD \rangle / \mu m^2$

Time Scale/s

*FIG. 1D*

FIG. 1E

FIG. 1F

FIG. 1G

*FIG. 1H*

*FIG. 2A*

FIG. 2B

FIG. 3A

FIG. 3B

FIG. 3C

FIG. 4A          FIG. 4B          FIG. 4C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6509323 B
- US 5034506 A
- US 20070219122 A
- US 2008050920 A
- US 20110262406 A
- US 7052678 B, Vanbever
- US 4765539 A, Noakes
- US 4962885 A, Coffee, R.A.

**Non-patent literature cited in the description**

- **GREF et al.** *Colloids and Surfaces B: Biointerfaces,* 2000, vol. 18, 301-313
- **FAHMY et al.** *Biomaterials,* 2005, vol. 26, 5727-5736
- **CU ; SALTZMAN.** *Mol. Pharm.,* 2009, vol. 6 (1), 173-181
- **SHENG et al.** *J. Mater. Sci: Mater Med,* 2009, vol. 20, 1881-1891
- *USP Bulk Density and Tapped Density, United States Pharmacopia convention,* 1999, 4950-4951
- Gene Delivery to the Lung. **HANES, J. et al.** Pharmaceutical Inhalation Aerosol Technology. Marcel Dekker, Inc, 2003, 489-539
- **GREGORY, R. J. et al.** *Nature,* 1990, vol. 347, 382386
- **RICH, D. P. et al.** *Nature,* 1990, vol. 347, 358-362
- **RIORDAN, J. R. et al.** *Science,* 1989, vol. 245, 1066-1073
- **STERCHAK, E. P. et al.** *Organic Chem.,* 1987, vol. 52, 4202
- **HU et al.** *Mol. Biotech.,* 2005, vol. 29, 197-210
- **OLSEN et al.** *J. Gene Med.,* 2005, vol. 7, 1534-1544
- **KAWAGUCHI, H. et al.** *Biomaterials,* 1986, vol. 7, 61-66
- **KRENIS, L. J. ; STRAUSS, B.** *Proc. Soc. Exp. Med.,* 1961, vol. 107, 748-750
- **RUDT, S. ; MULLER, R. H.** *J. Contr. Rel,* 1992, vol. 22, 263-272
- Handbook of Pharmaceutical Excipients. Pharmaceutical Press, 2003
- Pharmaceutical dosage form tablets. Marcel Dekker, Inc, 1989
- Remington - The science and practice of pharmacy. Lippincott Williams & Wilkins, 2000
- Pharmaceutical dosage forms and drug delivery systems. Williams and Wilkins, 1995
- **R. C. MUNDARGI et al.** *J. Control. Release,* 2008, vol. 125, 193
- **M. LI et al.** *Int. J. Pharm.,* 2008, vol. 363, 26
- **C. E. ASTETE ; C. M. SABLIOV.** *J. Biomater. Sci. Polymer Ed.,* 2006, vol. 17, 247
- **R. A. JAIN.** *Biomaterials,* 2000, vol. 21, 2475